(19) Europäisches Patentamt
European Patent Office
Office européen des brevets

(11) **EP 4 674 412 A1**

(12) **EUROPEAN PATENT APPLICATION**
published in accordance with Art. 153(4) EPC

(43) Date of publication:
07.01.2026 Bulletin 2026/02

(21) Application number: 25742102.4

(22) Date of filing: 15.01.2025

(51) International Patent Classification (IPC):
*A61K 9/51* (2006.01)    *A61K 31/7088* (2006.01)
*A61K 31/7105* (2006.01)    *A61K 9/1272* (2025.01)
*B01L 3/00* (2006.01)    *B01J 19/00* (2006.01)

(52) Cooperative Patent Classification (CPC):
A61K 9/127; A61K 9/1272; A61K 9/51;
A61K 31/7088; A61K 31/7105; B01J 19/00;
B01L 3/00

(86) International application number:
PCT/KR2025/000889

(87) International publication number:
WO 2025/155087 (24.07.2025 Gazette 2025/30)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 19.01.2024 KR 20240009087

(71) Applicant: Mepsgen Co., Ltd.
Seoul 05836 (KR)

(72) Inventors:
• JEON, Mi Yeon
Seoul 05667 (KR)
• LEE, Woo Seung
Seoul 05667 (KR)
• KIM, Se Yeon
Seoul 05518 (KR)
• KIM, Yong Tae
Seoul 06091 (KR)

(74) Representative: IPrime Rentsch Kaelin AG
Hirschengraben 1
8001 Zürich (CH)

(54) **MICROFLUIDIC DEVICE FOR PREPARING LIPID NANOPARTICLES OF VARIOUS SIZES, AND PREPARATION METHOD USING SAME**

(57) The present invention relates to a microfluidic device for preparing lipid nanoparticles capable of delivering nucleic acids, and a method for preparing lipid nanoparticles using the same. Using the microfluidic device, lipid nanoparticles having a desired size can be prepared by adjusting the molar ratio of compositions and the Reynolds number.

[FIG. 1]

**EP 4 674 412 A1**

**Description**

[Technical Field]

The present invention relates to a microfluidic device for preparing lipid nanoparticles for nucleic acid delivery, and a method for preparing lipid nanoparticles of various sizes using the same. Specifically, the present invention relates to a method for preparing lipid nanoparticles having a specific size by regulating a composition ratio of sterols and constitutional components constituting the lipid nanoparticles and adjusting the Reynolds number, and a microfluidic device capable of efficient mixing in the production of lipid nanoparticles, thereby enabling mass production of lipid nanoparticles with various sizes.

[Background Art]

The gene therapy market has been growing recently. Gene therapy is a drug that treats and prevents diseases by delivering genes to a disease site and producing proteins from the genes. Various gene therapy products with different mechanisms, from Pfizer and Moderna's COVID-19 vaccines to Biogen and Ionis' Qalsody, which is a therapeutic for Lou Gehrig's disease using RNA interference (RNAi), are either FDA-approved or undergoing clinical trials.

It is important for these gene therapies to accurately deliver genes that are easily destroyed in the human body to the lesion, and various studies are being conducted to increase the efficiency of delivery in the human body. Gene therapies using various types of vectors are being studied, and since vectors alone still have a high likelihood of being destroyed in the human body, lipid nanoparticles (LNPs) are also being actively studied to safely protect and deliver them.

Lipid nanoparticles (LNPs) are nanoparticles composed of lipids such as phospholipids and cholesterol, and they protect carriers from destruction in the human body and neutralize the charge of the carriers to increase cell-membrane penetration efficiency. They are also effective in drug delivery of modalities other than genes, and many pharmaceutical companies are conducting research on new lipid nanoparticles. However, the synthesis and manufacturing of these lipid nanoparticles have the problem that nanoparticles having different sizes such as micelles and liposomes can be formed because it is difficult to control phospholipids and cholesterol, which spontaneously aggregate and interact.

Cholesterol in lipid nanoparticles is known to affect biomembranes composed of phospholipids in various ways. In general, as the composition ratio of cholesterol included in the biomembrane increases, the fluidity of the biomembrane decreases and the binding between phospholipids becomes stronger. In lipid nanoparticles intended for the delivery of small-molecule drugs, it has been confirmed by researchers that lipid nanoparticles with a high cholesterol composition ratio exhibit improved drug loading efficiency and particle stability. For this reason, lipid nanoparticles for nucleic acid delivery have generally included cholesterol as a component. However, as mentioned above, including cholesterol makes it difficult to produce uniform lipid nanoparticles, and in reality, it is quite challenging to encapsulate gene therapeutics at a high level while controlling their size.

The COVID-19 vaccine is currently the most commercialized lipid nanoparticle-based vaccine. The messenger RNA (mRNA) used therein generally has a sequence length of thousands of bases. Typically, the length of mRNA used as a therapeutic is known to be 1,000 to 5,000 nt. In contrast, antisense oligonucleotides (ASOs), which are only several tens of bases in length, are gene therapeutics based on inhibiting RNA expression, and therapeutic development using lipid nanoparticles is also underway for these molecules. In technologies utilizing lipid nanoparticles as carriers for nucleic acids, different levels of leakage occur depending on the sequence length of the nucleic acids used, and therefore, the composition ratio of cholesterol needs to be adjusted according to the length of the encapsulated nucleic acids. That is, in the case of nucleic acids with relatively long sequences (e.g., mRNA), the typical composition ratio of lipid nanoparticles, i.e., an equimolar ratio of phospholipids to cholesterol, means an unnecessarily excessive cholesterol content. On the other hand, nucleic acids with relatively short sequences (e.g., oligonucleotides) exhibit relatively high leakage from lipid nanoparticles, and thus require a higher cholesterol content.

The present inventors have conducted various studies on the production of lipid nanoparticles having different composition ratios, and consequently, have devised a method for preparing lipid nanoparticles by adjusting molar ratios of lipid nanoparticle compositions and Reynolds numbers, as well as a method for producing the same with high efficiency in a separately designed expandable microfluidic device, and have thereby completed the present invention by elucidating the effects thereof.

(Prior Art Document)

(Patent Document)

(Patent Document 1) US Patent No. US8058069B2

(Non-Patent Document)

(Non-Patent Document 1) Camilla, H., A. et al. The role of lipid components in lipid nanoparticles for vaccines and gene therapy. Adv. Drug Delivery. Rev., 188, 114416 (2022).
(Non-Patent Document 2) Cheng, Q. et al. Selective organ targeting (SORT) nanoparticles for tissue-specific mRNA delivery and CRISPR-Cas gene editing. Nat. Nanotech., 15(4), 313-320 (2020).

[Summary of Invention]

[Problems to be Solved by Invention]

As mentioned above, lipid nanoparticles have the problem that it is very difficult to regulate the particle size due to various byproducts formed from different lipids.

An object of the present invention is to provide a preparation method for regulating a molar ratio between components in lipid nanoparticles for the purpose of nucleic acid delivery, and for controlling the particle size that varies according to the molar ratio and the Reynolds number.

Another object of the present invention is to provide a preparation method that improves the efficiency of lipid nanoparticle production by applying the preparation method to a separately designed microfluidic device.

[Means for Solving Problems]

The present invention provides a method for preparing lipid nanoparticles in a microfluidic device including an inlet channel, a mixing channel, and an outlet channel, which includes:

(a) injecting a lipid mixture into the inlet channel;
(b) injecting nucleic acid into another inlet channel different from the inlet channel, and
(c) preparing lipid nanoparticles by mixing a lipid mixture and nucleic acid in the mixing channel.

The mixing channel may include microposts.

In the microfluidic device, the number of the inlet channels may be two or more, and the flow directions of fluids introduced through different inlet channels may be different from each other.

The lipid mixture may include, but is not limited to, one or more selected from the group consisting of an ionizable lipid, a helper lipid, a PEGylated lipid, and a sterol.

The ionizable lipid may include at least one selected from the group consisting of: 9-heptadecanyl 8-{(2-hydroxyethyl)[6-oxo-6-(undecyclooxy)hexyl]amino}octanoate (SM-102); 1,2-dilinoleyloxy-N,N-dimethyl-3-aminopropane (DLinDMA); 2,2-dilinoleyl-4-dimethylaminoethyl-[1,3]-dioxolane (DLin-KC2-DMA); (6Z,9Z,28Z,31Z)-heptatriaconta-6,9,28,31-tetraen-19-yl 4-(dimethylamino)butanoate (DLin-MC3-DMA); 6-((2-hexyldecanoyl)oxy)-N-(6-((2-hexyldecanoyl)oxy)hexyl)-N-(4-hydroxybutyl)hexan-1-aminum (ALC-0315); 1,2-dioleyloxy-3-dimethylaminopropane (DODMA); and 1,2-dioleyl-3-dimethylammonium-propane (DODAP), but is not limited thereto.

In one embodiment, the ionizable lipid may be 9-heptadecanyl 8-{(2-hydroxyethyl)[6-oxo-6-(undecyclooxy)hexyl]amino}octanoate (SM-102).

The helper lipid may include at least one selected from the group consisting of: distearoylphosphatidylcholine (DSPC); palmitoyloleoylphosphatidylcholine (POPC); 1,2-dioleoyl-sn-glycero-3-phosphate (18:1 PA); dipalmitoylphosphatidylcholine (DPPC); dioleoylphosphatidylcholine (DOPC); dioleoylphosphatidylglycerol (DOPG); dipalmitoylphosphatidylglycerol (DPPG); dioleoylphosphatidylethanolamine (DOPE); palmitoyloleoylphosphatidylethanolamine (POPE); dioleoyl-phosphatidylethanolamine 4-(N-maleimidomethyl)-cyclohexane-1-carboxylate (DOPE-mal); dipalmitoylphosphatidylethanolamine (DPPE); 1,2-dimyristoyl-sn-glycero-3-phosphocholine (DMPC); sphingomyelin (SM), but is not limited thereto.

In one embodiment, the helper lipid may be distearoylphosphatidylcholine (DSPC) or palmitoyloleoylphosphatidylcholine (POPC).

The PEGylated lipid may include at least one selected from the group consisting of: 1,2-dimyristoyl-rac-glycerol methoxypolyethylene glycol (DMG-PEG); polyethylene glycol-diacylglycerol (PEG-DAG); polyethylene glycol-dialkyloxypropyl (PEG-DAA); polyethylene glycol-dimethacrylate (PEG-DMA); distearoyl-rac-glycerol methoxypolyethylene glycol (DSG-PEG); polyethylene glycol-ceramide (PEG-Ceramide); polyethylene glycol-phospholipid (PEG-phospholipid); polyethylene glycol-phosphatidylethanolamine (PEG-PE); polyethylene glycol-succinoyl 1,2-diacylglycerol (PEG-S-DAG); polyethylene glycol-dialkyloxypropylcarbamate (PEG-dialkyloxypropylcarbamate), but is not limited thereto.

In one embodiment, the PEGylated lipid may be 1,2-dimyristoyl-rac-glycerol methoxypolyethylene glycol (DMG-PEG2k).

The sterols may include at least one selected from the group consisting of cholesterol, ergosterol, campesterol, stigmasterol, sitosterol, fucosterol, betulin, lupeol, ursolic acid, oleanolic acid, brassicasterol, 9,11-dehydroergosterol, daucosterol, β-sitosterol-acetate, β-sitosterol-amino acid conjugates, 20-hydroxycholesterol, 22-hydroxycholesterol, 24-hydroxycholesterol, 25-hydroxycholesterol, and 27-hydroxycholesterol, but is not limited thereto.

In one embodiment, the sterol may be cholesterol.

The nucleic acid may include at least one selected from the group consisting of mRNA, siRNA, aiRNA, miRNA, dsRNA, shRNA, lncRNA, saRNA, rRNA, tRNA, piRNA, circRNA, RNA, DNA, cDNA, plasmid, aptamer, oligonucleotide, ribozyme, PNA, and DNAzyme, but is not limited thereto.

In one embodiment, the nucleic acid may be mRNA or an oligonucleotide.

A polydispersity index (PDI) of the lipid nanoparticles may be 0.3 or less, and preferably 0.2 or less.

A nucleic acid encapsulation efficiency of the lipid nanoparticles may be 50% or more, and preferably 70% or more.

A nucleic acid loading efficiency of the lipid nanoparticles may be 15% or more, and preferably 20% or more.

A size of the lipid nanoparticles may be adjusted according to the Reynolds number for the fluid flow in the mixing channel, and the Reynolds number may be 10 to 150, preferably 25 to 100.

The lipid nanoparticles may have a size of 10 nm to 200 nm, preferably 20 nm to 150 nm, and most preferably 30 nm to 90 nm.

The size of the lipid nanoparticles may be adjusted according to the molar ratio of the lipid mixture and the sterol. The molar ratio of the lipid mixture to the sterol may be 1:0.1 or more, and preferably 1:0.3 or more.

The present invention provides lipid nanoparticles obtained by the method for preparing lipid nanoparticles.

The present invention provides a microfluidic device for preparing lipid nanoparticles, including an inlet channel, a mixing channel and an outlet channel,

> the number of the inlet channels is two or more,
> the mixing channel includes microposts, and flow directions of fluids flowing into different inlet channels are different from each other.

A fluid including a lipid mixture and nucleic acids may be introduced into the microfluidic device through the inlet channel, and the lipid mixture may include at least one selected from the group consisting of ionizable lipids, helper lipids, PEGylated lipids, and sterols, but is not limited thereto.

The lipid mixture may be introduced into one inlet channel, and the nucleic acids may be introduced into another inlet channel.

The lipid mixture may be introduced in the same direction as the fluid flow, and the nucleic acids may be introduced in a direction different from the fluid flow, and preferably the nucleic acids may be introduced in a direction perpendicular to the fluid flow.

The microposts may or may not be connected to the walls forming the mixing channel.

The micropost may be arranged in one or more rows in the direction of the fluid flow, and in one row, one or a plurality of microposts may be arranged in a direction different from the direction of the fluid flow.

The plurality of microposts may not be arranged in a single row in the direction of the fluid flow, and the plurality of microposts may be arranged so as to partially or completely cover the gaps between microposts arranged in adjacent rows when viewed in the direction of the fluid flow.

The size of the lipid nanoparticles may be adjusted according to the Reynolds number for the fluid flow in the mixing channel, and the Reynolds number may be 10 to 150, and preferably 25 to 100.

The size of the lipid nanoparticles may be adjusted by the molar ratio of the lipid mixture and the sterol. The molar ratio of the lipid mixture and the sterol may be 1:0.1 or more, and preferably 1:0.3 or more.

The polydispersity index (PDI) of the lipid nanoparticles may be 0.3 or less, and preferably 0.2 or less.

The nucleic acid encapsulation efficiency of the lipid nanoparticles may be 50% or more, and preferably 70% or more.

The nucleic acid loading efficiency of the lipid nanoparticles may be 15% or more, and preferably 20% or more.

[Advantageous Effects]

The method for preparing lipid nanoparticles of the present invention enables control of the particle size of lipid nanoparticles by adjusting the composition ratio of raw materials including cholesterol. The method for preparing lipid nanoparticles of the present invention may be used in the production of pharmaceuticals containing lipid nanoparticles.

In addition, by applying the lipid nanoparticle preparation method of the present invention to a manufacturing process for pharmaceuticals using nucleic acids as a modality, messenger ribonucleic acid may be encapsulated in lipid nanoparticles of various sizes at a high concentration, thereby enhancing therapeutic efficiency

[Brief Description of Drawings]

FIG. 1 is a schematic diagram illustrating the synthesis process of lipid nanoparticles (LNPs) containing nucleic acids using a micro mixing channel device of the present invention and the morphology of nucleic acid-containing LNPs.

FIG. 2 is a schematic diagram showing the length of each section of the device for the structure and design of the device of the present invention.

FIG. 3 shows the fluid flow through the device of the present invention and the fluid flow around the microposts.

FIG. 4 shows the results of measuring the mixing efficiency according to the number of micropost rows in the device of the present invention.

FIG. 5 shows the results of measuring the mixing efficiency according to the number of microposts present in one row in the device of the present invention.

FIG. 6 shows the result of a Dean vortex formed by the width gap of microposts arranged in a mixing zone in the device of the present invention.

FIG. 7 shows the results of the Dean number and the mixing efficiency according to the width gap of microposts arranged in the mixing zone in the device of the present invention.

FIG. 8 shows the results of measuring the mixing efficiency according to changes in the Reynolds number in the device of the present invention.

FIG. 9 shows the results of measuring the mixing efficiency according to changes in the channel height in the device of the present invention.

FIG. 10 shows the results of measuring the mixing efficiency according to the injection ratio of the aqueous solution and the lipid mixture in the device of the present invention.

FIG. 11 illustrates a calculation method for the residence time of a fluid.

FIG. 12 shows the fluid flow within the device according to Preparative Examples 1 and 2 of the present invention.

FIG. 13 shows the results of measuring different mixing efficiencies according to changes in the micropost initial spacing, spacing between microposts, and longitudinal length of microposts, respectively, in the device of the present invention.

FIG. 14 shows the result of measuring the mixing efficiency to changes in blockage rate in the device of the present invention.

FIG. 15 shows the result of mixing efficiency within the device according to the front and rear width gap by the microposts in the device of the present invention.

FIG. 16 shows the result of shear rate to Reynolds number at each width gap by the microposts in the device of the present invention.

FIG. 17 shows the result of design conditions to size for the device of the present invention.

FIG. 18 shows the results of mixing efficiency to Reynolds number by the device size of the present invention.

FIG. 19 shows the results of flow rate to Reynolds number by the device size of the present invention.

FIG. 20 shows the results of shear rate to Reynolds number by the device size of the present invention.

FIG. 21 is a graph comparing the size and polydispersity index (PDI) of LNPs according to Reynolds number when the composition ratio (molar ratio) of SM-102:DSPC/POPC:Cholesterol:DMG-PEG2k is 50:10:38.5:1.5.

FIGS. 22 and 23 show the DLS results for the size of LNPs according to the Reynolds number of FIG. 21.

FIG. 24 is a graph comparing the size and polydispersity index (PDI) of LNPs according to Reynolds number when the composition ratio (molar ratio) of SM-102:DSPC/POPC:Cholesterol:DMG-PEG2k is 28:5.7:65.5:0.8.

FIGS. 25 and 26 show the DLS results for the size of LNPs according to the Reynolds number of FIG. 24.

FIG. 27 is a graph comparing the size and polydispersity index (PDI) of LNPs according to Reynolds number when the composition ratio (molar ratio) of SM-102:DSPC/POPC:Cholesterol:DMG-PEG2k is 20:4:75.5:0.5.

FIGS. 28 and 29 show the DLS results for the size of LNPs according to the Reynolds number of FIG. 27.

FIG. 30 is a graph comparing the size and polydispersity index (PDI) of LNPs according to the cholesterol molar ratio.

FIGS. 31 and 32 are number distributions of the DLS results for the size of LNPs according to the cholesterol molar ratio of FIG. 30.

FIGS. 33 and 34 are volume distributions of the DLS results for the size of LNPs according to the cholesterol molar ratio of FIG. 30.

FIGS. 35 and 36 are intensity distributions of the DLS results for the size of LNPs according to the cholesterol molar ratio of FIG. 30.

FIG. 37 is a graph comparing the size and polydispersity index (PDI) of LNPs at Reynolds numbers of 50 and 100 when the composition ratio (molar ratio) of SM-102:DSPC/POPC:Cholesterol:DMG-PEG2k is 48.5:9.5:37.5:4.5.

FIG. 38 shows the DLS results for the size of LNPs according to the Reynolds number of FIG. 37.

FIG. 39 is a graph comparing the size and PDI of Bare LNPs without mRNA and mCherry LNPs under synthetic conditions with a size of 30 nm.

FIG. 40 is a graph comparing the size and PDI of Bare LNPs without mRNA and mCherry LNPs under synthetic conditions with a size of 60 nm.

FIG. 41 is a graph comparing the size and PDI of Bare LNPs without mRNA and mCherry LNPs under synthetic conditions with a size of 90 nm.

FIGS. 42 and 43 show the DLS results for the sizes of Bare LNPs and mCherry LNPs without mRNA in FIGS. 39, 40 and 41.

FIGS. 44 and 45 are graphs comparing the size and PDI of Bare LNPs without mRNA and Cas9 LNPs under synthetic conditions with a size of 30 nm.

FIG. 46 is a graph comparing the size and PDI of Bare LNPs without mRNA and Cas9 LNPs under synthetic conditions with a size of 60 nm.

FIG. 47 is a graph comparing the size and PDI of Bare LNPs without mRNA and Cas9 LNPs under synthetic conditions with a size of 90 nm.

FIGS. 48 and 49 show the DLS results for the sizes of Bare LNPs and Cas9 LNPs without mRNA in FIGS. 44, 45, 46, and 47.

FIG. 50 is a graph comparing the encapsulation efficiency (EE) of mCherry LNPs under synthetic conditions with sizes of 30 nm, 60 nm, and 90 nm.

FIG. 51 is a graph comparing the encapsulation efficiency (EE) of Cas9 LNPs under synthetic conditions with sizes of 30 nm, 60 nm, and 90 nm.

FIG. 52 is a graph comparing the size and PDI of Bare LNPs without mRNA and Oligo LNPs under synthetic conditions with a size of 30 nm.

FIG. 53 is a graph comparing the size and PDI of Bare LNPs without mRNA and Oligo LNPs under synthetic conditions with a size of 60 nm.

FIG. 54 is a graph comparing the size and PDI of Bare LNPs without mRNA and Oligo LNPs under synthetic conditions with a size of 90 nm.

FIGS. 55 and 56 show the DLS results for the sizes of Bare LNPs and Oligo LNPs without mRNA in FIGS. 52, 53, and 54.

FIG. 57 is a graph comparing the encapsulation efficiency (EE) of Oligo LNPs under synthetic conditions with sizes of 30 nm, 60 nm, and 90 nm.

FIG. 58 is a fluorescence image showing the level of mCherry protein expression after treatment with mCherry LNPs (200 ng of mCherry mRNA) under synthetic conditions with sizes of 30 nm, 60 nm, and 90 nm in human glioblastoma U87MG cells.

FIG. 59 is a graph quantifying the fluorescence of mCherry protein in the U87MG fluorescence image of FIG. 58.

FIG. 60 is a fluorescence image showing the level of mCherry protein expression according to the amount of mCherry mRNA (500, 200, and 20 ng) under synthetic conditions with sizes of 60 nm and 90 nm in the human hepatoma cell line HepG2.

FIG. 61 is a graph quantifying the fluorescence of mCherry protein in the HepG2 fluorescence image of FIG. 60.

FIG. 62 is a fluorescence image showing the level of mCherry protein expression to the amount of mCherry mRNA processed (500, 200, 20 ng) under synthetic conditions of 60 nm and 90 nm in size in the human monocyte cell line THP-1.

FIG. 63 is a graph for quantification of the fluorescence of mCherry protein in the THP-1 fluorescence image of FIG. 62.

FIGS. 64 and 65 show the results of size, polydispersity index (PDI), mCherry mRNA encapsulation efficiency (EE), and loading efficiency (LE) for storage stability after synthesis of 60 nm mCherry LNPs.

FIGS. 66 and 67 show the results of size, polydispersity index (PDI), mCherry mRNA encapsulation efficiency (EE), and loading efficiency (LE) for storage stability after synthesis of 90 nm mCherry LNPs.

FIGS. 68 and 69 show the results of size, polydispersity index (PDI), Cas9 mRNA encapsulation efficiency (EE), and loading efficiency (LE) for storage stability after synthesis of 30 nm Cas9 LNP.

FIGS. 70 and 71 show the results of size, polydispersity index (PDI), Cas9 mRNA encapsulation efficiency (EE), and loading efficiency (LE) for storage stability after synthesis of 90 nm Cas9 LNP.

FIGS. 72 and 73 show the results of size, polydispersity index (PDI), oligonucleotide encapsulation efficiency (EE), and loading efficiency (LE) for storage stability after synthesis of 50 nm Oligo LNPs (target size: 30 nm).

FIGS. 74 and 75 show the results of size, polydispersity index (PDI), oligonucleotide encapsulation efficiency (EE), and loading efficiency (LE) for storage stability after synthesis of 100 nm Oligo LNPs (target size: 60 nm).

FIGS. 76 and 77 show the results of size, polydispersity index (PDI), oligonucleotide encapsulation efficiency (EE), and loading efficiency (LE) for storage stability after synthesis of 130 nm Oligo LNPs (target size: 90 nm).

[Mode for Carrying out Invention]

Hereinafter, embodiments and examples of the present invention will be described in detail with reference to the accompanying drawings, so that those skilled in the art can readily practice the present invention. However, it should be understood that the present invention may be embodied in various forms and is not limited to the specific embodiments and examples described herein.

Throughout this specification, when a part is referred to as "including" a component, it should be understood that, unless explicitly stated otherwise, the term does not exclude the presence of other components but may further include additional components.

The term "lipid nanoparticle" as used herein refers to a particle including four types of components: ionizable lipid, helper lipid, PEGylated lipid, and sterol, wherein the helper lipid may include one or more types of lipids. It also refers to a nano-sized particle capable of encapsulating nucleic acids therein.

As used herein, the term "microfluidic device" refers to a device including a microchannel configured to allow a fluid to flow on a substrate made of various materials, including plastic, glass, metal, or silicon, and which may include an organic polymer.

The inlet channel, mixing channel and outlet channel may be arranged sequentially in the direction of fluid flow.

The inlet channel may include a channel for introducing a nucleic acid and a channel for introducing a lipid mixture, and the nucleic acid and the lipid mixture may be introduced through different inlet channels, respectively. In this regard, the lipid mixture may be introduced in the same direction as the fluid flow in the mixing channel, and the nucleic acid may be introduced in a direction different from the fluid flow in the mixing channel. Preferably, the lipid mixture may be introduced in the same direction as the fluid flow, and the nucleic acid may be introduced in a direction perpendicular to the fluid flow.

The inlet channel into which the lipid mixture is introduced may be one or more, and the inlet channel into which the nucleic acid is introduced may be one or more. Preferably, the device of the present invention may include one inlet channel into which the lipid mixture is introduced and two inlet channels into which the nucleic acid is introduced.

In the mixing channel, the nucleic acid and lipid mixture may be mixed with each other.

A size of the particle may be adjusted by changing the Reynolds number for the fluid flow in the mixing channel. Further, the Reynolds number for the fluid flow in the mixing channel may be 10 to 150, and preferably 25 to 100.

The channel may have a height of 200 to 800 $\mu$m.

The term "fluid flow" as used herein refers to a direction in which fluid flows from the inlet channel to the outlet channel within the device. Preferably, the lipid mixture is introduced through the inlet channel arranged in the same direction as the flow direction in the mixing channel, and the nucleic acids are introduced through the inlet channel arranged in a direction perpendicular to the flow direction in the mixing channel.

In general, the flow rate within a device may be expressed by the Reynolds number (Re), which is a dimensionless number representing the ratio of inertial force to viscous force, and the calculation formula is as follows:

$$Re = \frac{\rho v D_h}{\mu} = \frac{\rho v}{\mu} \frac{2wh}{w+h} = \frac{\rho}{\mu} \frac{2Q}{w+h}$$

Herein, $D_h$ is the hydraulic diameter within the microfluidic device, and $\rho$, $\mu$, $v$ and $Q$ represent the density, dynamic viscosity, velocity, and flow rate of the fluid, respectively. Therefore, as the Reynolds number increases, the flow rate increases proportionally. When the Reynolds number exceeds 500, a turbulence-like flow is formed within the device, so that a controllable vortex pattern is not generated, and an increase in shear force between the solvent and the substance due to the high flow rate may cause instability of the raw drug substance and the generated nanoparticles. When the Reynolds number is 300 or more, uncontrollable chaotic flow begins to occur within the device, making quality control for producing uniform nanoparticles difficult. On the other hand, when the Reynolds number is as low as 10 or less, the process becomes dependent on diffusion, making it difficult to achieve effective mixing of the lipid mixture and nucleic acid, and thus difficult to obtain superior productivity compared with that of conventional nanoparticle synthesis methods.

The device of the present invention may regulate the particle size by changing the Reynolds number.

The term "micropost" as used herein refers to a structure that disrupts the straight flow of fluid within a mixing channel. The micropost of the present invention is a structure for efficiently mixing fluids introduced into the device by forming microvortices, and may include any shape, and is preferably a columnar shape. The micropost may be designed to separate or bend the flow of fluids striking the micropost, or to merge them with different flows. The micropost may be designed to maintain the main flow of the fluid and prevent the flow from stagnating, and may, for example, intersect the fluid at a right angle with respect to the direction of flow of the fluid.

The micropost may or may not be connected to a wall forming the mixing channel, and may be at least one selected from the group consisting of polyhedra, truncated polyhedra, polygonal columns, and modified shapes thereof, and preferably may be a square column.

The microposts may be arranged in one or more rows in the direction of the fluid flow, and preferably in six or more rows in the direction of the fluid flow. Further, one or more microposts may be arranged in a direction different from the direction of the fluid flow in one row, and preferably 1 to 6 microposts may be arranged in a direction different from the direction of the fluid flow in one row, more preferably 1 to 6 microposts may be arranged in a direction perpendicular to the direction of the fluid flow in one row, and most preferably 1 to 2 microposts may be arranged in a direction perpendicular to the direction of the fluid flow in one row.

The plurality of microposts may not be arranged in a single row in the direction of the fluid flow, and the plurality of microposts may be arranged so as to partially or completely cover the gaps between microposts arranged in adjacent rows when viewed in the direction of the fluid flow.

In the present invention, the microposts may be arranged in an alternating manner with respect to the microposts in adjacent rows. As used herein, the term "arranged in an alternating manner" means that the microposts in the plurality of rows are not aligned in a straight line or arranged in a single parallel row in the flow direction of the fluid. Instead, the microposts arranged in one row may be positioned to partially or completely block the gaps between the microposts arranged in adjacent rows when viewed in the flow direction of the fluid.

In the present invention, the micropost may have a height of 200 to 800 $\mu$m, and the micropost may or may not be connected to the walls forming the mixing channel.

The term "mixing efficiency (*Mixeff*)" as used herein refers to a value expressed as a percentage, calculated based on the principle that the lipid mixture and nucleic acid, each having a mass fraction of 1, mix with each other in the channel and ultimately converge to 0.5. The calculation formula is as follows.

$$Mix_{eff} = \begin{cases} 2 \cdot Mass_{fract} & Mass_{fract} \leq 0.5 \\ 2(1 - Mass_{fract}) & Mass_{fract} > 0.5 \end{cases}$$

At this time, Mixeff represents the mass fraction, and the closer the mass fraction is to 0.5, the higher the mixing efficiency proportionally.

The outlet channel may be a channel through which the generated lipid nanoparticles outflow.

The term "inlet volume flow rate ratio" as used herein refers to the ratio of the inlet volume flow rate of an aqueous fluid to the inlet volume flow rate of a fluid containing a lipid mixture.

In the present invention, the term "flow blockage ratio" refers to the ratio of the width of the micropost to the width of the entire channel in the first row along the flow direction of the fluid in the mixing channel.

The term "nucleic acid encapsulation efficiency (EE)" as used herein refers to a value calculated from each mRNA LNP based on fluorescence intensity, by detecting the total mRNA and the unencapsulated mRNA (free mRNA). The value is calculated according to the following equation:

$$\text{Encapsulation Efficiency (\%)} = \frac{\text{Total mRNA} - \text{Free mRNA}}{\text{Total mRNA}} \times 100.$$

The term "Total mRNA" refers to the total amount of mRNA present in the final product of the lipid nanoparticle preparation process, and the term "Free mRNA" refers to the amount of mRNA present outside the lipid nanoparticles in the final product of the lipid nanoparticle preparation process.

The term "nucleic acid loading efficiency (LE)" as used refers to a value calculated from each mRNA LNP based on fluorescence intensity, by detecting the total mRNA and the unencapsulated mRNA (free mRNA). The value is calculated according to the following equation:

$$\text{Loading Efficiency (\%)} = \frac{\text{Total mRNA} - \text{Free mRNA}}{\text{Feeding mRNA}} \times 100.$$

The term "Feeding mRNA" refers to the total amount of mRNA introduced into the lipid nanoparticle preparation process.

The present invention will be described in more detail through the following examples; however, the following examples are for the purpose of description only and are not intended to limit the scope of the present invention.

**[Example 1]**

**Design of device for preparing lipid nanoparticles**

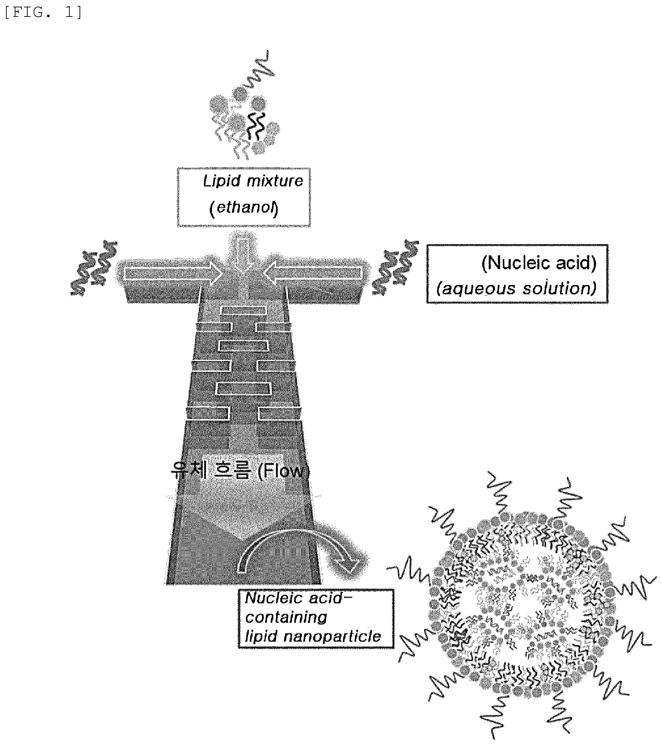

A microfluidic device was designed to prepare lipid nanoparticles (LNPs) (FIGS. 1, 2, and 3). The microfluidic device includes three inlet channels and one outlet channel. A lipid mixture was injected into the central inlet channel among the three inlet channels, and an aqueous solution containing nucleic acid was injected into the two inlet channels on both sides. In addition, a micropost structure was introduced inside the device to effectively mix the lipid mixture and nucleic acids.

**[Example 2]**

**Effect of single micropost structure**

To confirm the mixing efficiency of lipid mixtures and nucleic acid solutions according to the presence, number, and arrangement of microposts placed inside the device, the distribution of lipid mixtures inside the microfluidic device was analyzed using fluid dynamics techniques.

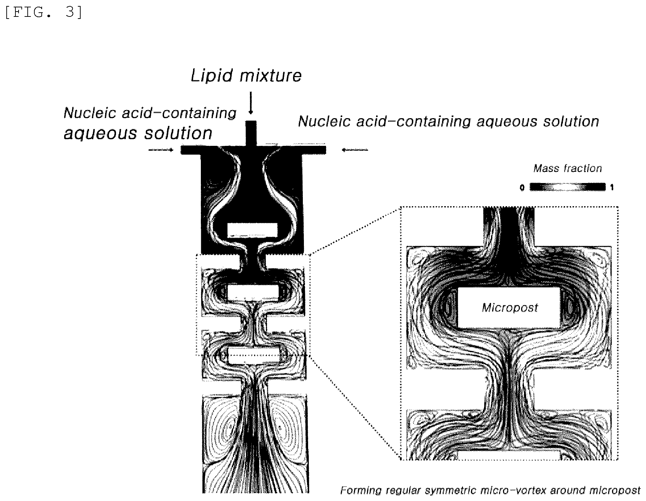

As shown in FIG. 3, it was confirmed that the lipid mixture and the nucleic acid solution were effectively mixed by microvortices generated as the lipid mixture flowed through the micropost structure.

In the present invention, the inlet volume flow rate ratio refers to the ratio of the inlet volume flow rate of the aqueous fluid to that of a fluid including a lipid mixture, and the flow blockage ratio is defined as the ratio of the width of a micropost to the width of the entire channel in the first row along the flow direction of the fluid in the mixing channel.

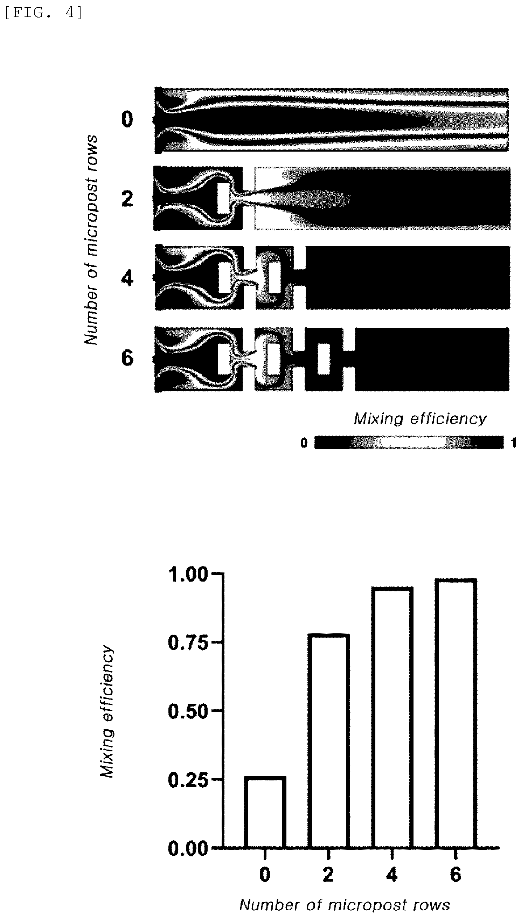

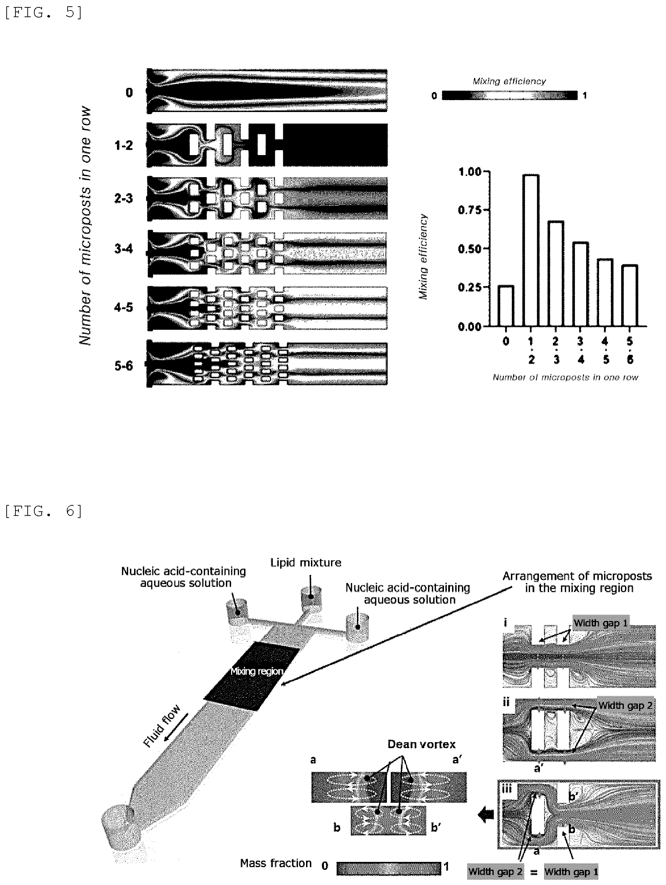

The results were verified under conditions including a channel height of 200 $\mu$m, an inlet volume flow rate ratio of 1:5.5 (lipid mixture:aqueous solution), a Reynolds number (Re) of 50, a flow blockage ratio of 0.5, and a rectangular micropost size of 1000 $\mu$m $\times$ 400 $\mu$m, as shown in FIGS. 4 and 5. The number of microposts was designed to be uniformly distributed across the channel width, while maintaining the size of the microposts and keeping constant the width of the flow path between the microposts.

As shown in FIGS. 4 and 5, it was confirmed that the mixing efficiency was improved by about 1.5 to 4.0 times depending on the number of microposts, in comparison with the case without microposts.

[TABLE 1]

| Mixing efficiency according to the number of micropost rows | |
| --- | --- |
| Number of micropost rows | Mixing efficiency |
| 0 | 0.26 |
| 2 | 0.78 |
| 4 | 0.95 |
| 6 | 0.98 |

As shown in FIG. 4 and Table 1 above, the mixing efficiency increases as the number of rows increases, and when the number is six or more, an excellent mixing efficiency of 98% or more is achieved.

[TABLE 2]

| Mixing efficiency according to the number of microposts in one row | |
| --- | --- |
| Number of microposts in one row | Mixing efficiency |
| 0 | 0.26 |
| 1-2 | 0.98 |
| 2-3 | 0.68 |
| 3-4 | 0.54 |

As shown in FIG. 5 and Table 2 above, as the number of microposts located within the channel in one row increases, the mixing efficiency decreases, and the best mixing efficiency is obtained when the number is 1 to 2.

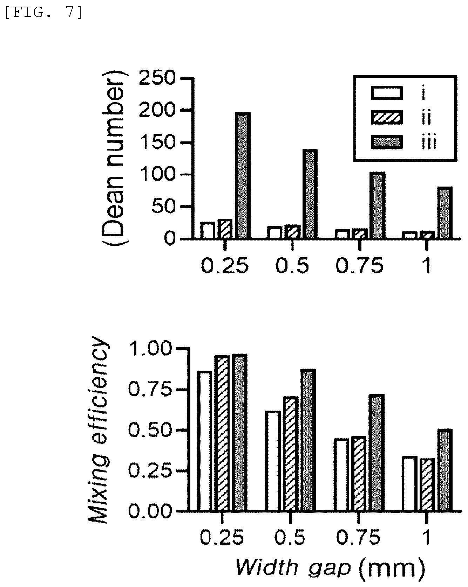

Therefore, the mixing efficiency varies depending on the number and arrangement of microposts inside the device. In particular, as shown in FIGS. 6 and 7, the highest mixing efficiency is obtained when a single micropost structure is arranged in a staggered manner. The microposts are arranged evenly with respect to the channel width, and the width gap through which the fluid passes between the microposts is kept constant.

**[Example 3]**

**Optimizing the available flow rate range and flow rate ratio range within the device**

To optimize LNP synthesis according to the flow rates and injection ratios of lipid mixture and aqueous solution within the device, the mixing efficiency was analyzed under various Reynolds number (Re) and flow rate ratio conditions.

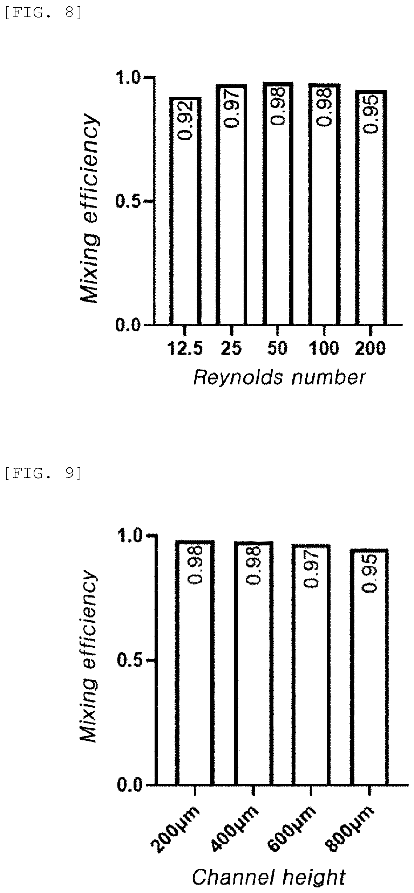

To investigate the available flow rate range, the mixing efficiency was analyzed when the Reynolds number (Re) was varied from 12.5 to 200 at a channel height of 200 $\mu$m and an injection ratio of 1:5.5 in the device, and the results are shown in FIG. 8 and Table 3.

[TABLE 3]

| Reynolds number | Mixing efficiency |
| --- | --- |
| 12.5 | 0.92 |
| 25 | 0.97 |
| 50 | 0.98 |
| 100 | 0.98 |
| 200 | 0.95 |

As shown in Table 3 above, a mixing efficiency of 90% or more was obtained when the Reynolds number (Re) was in the range of 12.5 to 200, and a mixing efficiency of 98% was obtained when the Reynolds number (Re) was 50 and 100.

Further, the mixing efficiency was analyzed when the channel height was varied from 200 to 800 $\mu$m at an injection ratio of 1:5.5 and a Reynolds number (Re) of 50, and the results are shown in FIG. 9 and Table 4.

[TABLE 4]

| Channel height ($\mu$m) | Mixing efficiency |
| --- | --- |
| 200 | 0.98 |
| 400 | 0.98 |
| 600 | 0.97 |
| 800 | 0.95 |

As shown in Table 4 above, a mixing efficiency of 95% or more was obtained when the channel height was in the range of 200 to 800 $\mu$m.

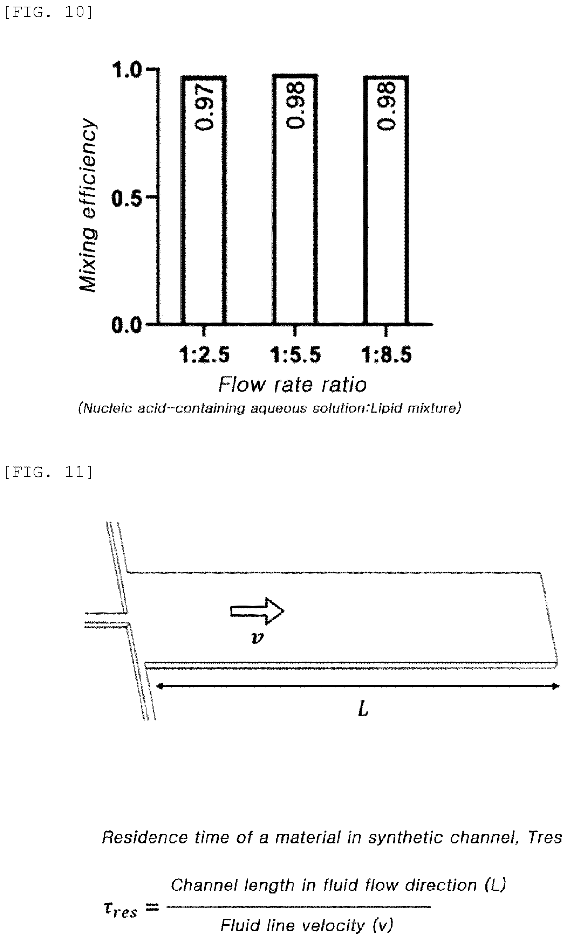

Further, the mixing efficiency was analyzed at a channel height of 200 $\mu$m and a Reynolds number (Re) of 50, while varying the injection ratio from 1:2.5 to 1:8.5, and the results are shown in FIG. 10 and Table 5.

[TABLE 5]

| Injection ratio | Mixing efficiency |
| --- | --- |
| 1:2.5 | 0.97 |
| 1:5.5 | 0.98 |
| 1:8.5 | 0.98 |

As shown in Table 5 above, a mixing efficiency of 97% or more was obtained when the injection ratio was in the range of 1:2.5 to 1:8.5.

**[Example 4]**

**Study on the height of the device for increasing mixing efficiency**

To maximize the mixing efficiency according to the height of the mixing channel of the device, the mixing efficiency was analyzed under different height conditions.

During the nanoparticle synthesis process using the device, when raw materials were injected at a relatively high flow rate (Re > 50), strong shear stress was generated from the wall due to the narrow channel width.

Organic substances in fluids have been reported to undergo aggregation and deformation when they exceed a certain value. In particular, in the case of proteins, many documents have reported that protein deformation, including aggregation, misfolding, and degradation, can be induced by the shear stress of fluid flow generated in external equipment. Such deformation of precursor proteins may change the structure, size, and even the function of the produced nanoparticles. In general, the range of deformation induced varies depending on the structure of the equipment that generates shear stress and the type and size of the material used. However, it has been reported that, in the case of proteins with a size of 1-10 nm (mass range of 20-300 kDa) such as insulin, enzymes, and immunoglobulins, the protein structure may be deformed at shear stress levels around 1000 dyne/cm$^2$ (Bekard, I., et al. The Effects of Shear Flow on Protein Structure and Function. Biopolymers, 95(11), 733-745 (2011)).

Therefore, in order to confirm whether precursor materials and nanoparticles are deformed by the shear stress ($\tau$w) generated through fluid flow in the device of the present invention, the shear stress generated according to each channel height was first calculated as follows.

$$\tau_w = \frac{6Q\mu}{h^2 w}$$

Herein, Q is the flow rate, and $\mu$, h and w represent the dynamic viscosity of the fluid, the height of the channel cross-section, and the width of the channel cross-section, respectively.

[TABLE 6]

| Channel height ($\mu$m) | Shear stress (dyne/cm$^2$), Re 50 |
|---|---|
| 100 | 165.00 |
| 200 | 41.25 |
| 400 | 10.31 |
| 800 | 2.58 |

As shown in Table 6 above, it was confirmed that as the channel height in the device of the present invention decreases, the shear stress increases exponentially, but remains below the threshold of 1000 dyne/cm$^2$ for protein structural deformation.

However, when the channel height was 100 $\mu$m, the shear stress reached the range of 1000 dyne/cm$^2$ when Re was increased to 300. Further, considering that the size of the nanoparticles to be produced is in the range of 10-100 nm, which is larger than that of proteins, it can be concluded that precursor materials and nanoparticles are not deformed by shear stress when the channel height is 200 $\mu$m or greater.

Further, as shown in Table 7 below, as the height of the mixing channel of the device increases, the volume increases, which in turn increases the residence time ($\tau_{res}$) of the mixed precursors in the channel at the same flow rate, and the mixing time ($\tau_{mix}$) required for synthesizing the precursors. A specific calculation method for the residence time of the mixed substances in the channel is shown in FIG. 11. The mixing time ($\tau_{mix}$) required for synthesizing the substances may be calculated according to the more dominant physical phenomenon-either convection or diffusion-in the device, and may be obtained as follows using the Peclet number (Pe), a dimensionless number representing the ratio of convection time to diffusion time.

$$\text{Pe} = \frac{t_{diffusion}}{t_{convection}}$$

In the device of the present invention, the Peclet number (Pe) is in the range of 100 to 5000, and based on this, it was confirmed that convective mixing is dominant. Therefore, the convective mixing time within the channel ($T_{mix, convection}$) was calculated as follows (Velencia, P. et al. Single-Step Assembly of Homogenous Lipid-Polymeric and Lipid-Quantum Dot Nanoparticles Enabled by Microfluidic Rapid Mixing. ACS Nano 4, 3, 1671-1679 (2010), and Rhee, M. et al. Drop Mixing in a Microchannel for Lab-on-a-Chip Platforms. Langmuir, 24 (2), 590-601 (2008)).

$$\tau_{mix,convection} \sim 0.286 \times \frac{w^2}{D} \times \left(\frac{Pe}{L/w}\right)^{-\frac{2}{3}}$$

Herein, D represents the diffusion coefficient, and L and w represent the length of the channel and the width of the channel cross-section, respectively.

[TABLE 7]

| Channel height ($\mu$m) | Residence time (ms) Mixing time (ms) | |
|---|---|---|
| | Re 50 | |
| 100 | 36 | 13 |
| 200 | 72 | 21 |
| 400 | 145 | 34 |
| 800 | 290 | 53 |

At this time, the residence time ($\tau_{res}$) must be longer than the mixing time ($\tau_{mix}$) so that the materials may be sufficiently mixed within the channel. As shown in Table 7 above, since the residence time is longer than the mixing time at all channel heights, it can be confirmed that sufficient mixing time is secured at a given channel height.

Further, the increase in mixing time according to the increase in channel height was calculated to be 53 ms at the maximum height of 800 $\mu$m as shown in Table 7 above, which falls within the aggregation time ($\tau_{agg}$) range of 50 to 100 ms during which the injected materials start to clump and aggregate. Therefore, it may be regarded as the maximum channel height applicable to the device and production method of the present invention (see Rohit, K. et al. Microfluidic Platform for Controlled Synthesis of Polymeric Nanoparticles. Nano Letters, 8, 9, 2906-12, (2008) and Johnson, B.K. et al. Mechanism for Rapid Self-Assembly of Block Copolymer Nanoparticles. Physical Review Letters, 91(11), 118302-1-4 (2003)).

Therefore, as shown in Table 7 above, it may be seen that the optimal height of the mixing channel that can prevent agglomeration and non-uniform nanoparticle generation of the obtained nanoparticles within the device is 200 to 800 $\mu$m.

**[Example 5]**

**Fluid flow within the device**

According to the channel height and micro-pillar conditions, the device of the present invention was designed in two preparative examples as shown in Table 8 below.

[TABLE 8]

| | | Preparative Example 1 | Preparative Example 2 |
|---|---|---|---|
| Channel | Height ($\mu$m) | 200 | 200 |
| Micropost | Horizontal x vertical size ($\mu$m) | 200 x 400 | 1000 x 400 |
| | Number of rows | 6 | 6 |
| | Number of microposts in one row | 5-6 | 1-2 |

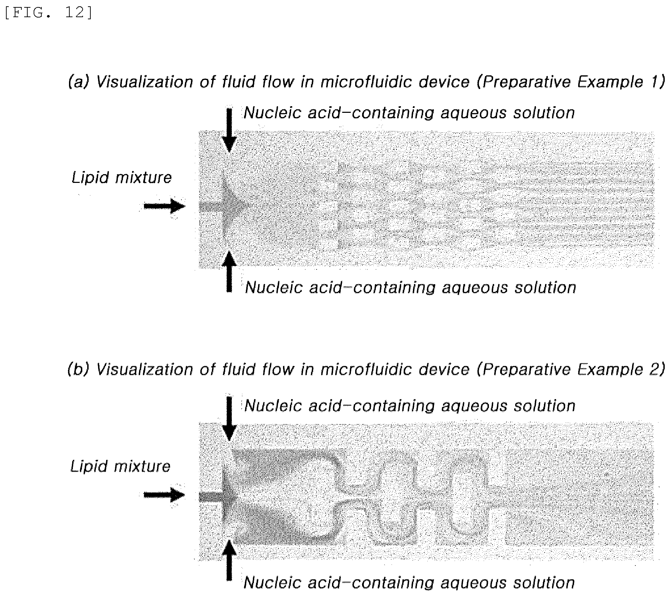

To produce Preparative Examples 1 and 2 and confirm the mixing flow pattern of the lipid mixture and the aqueous solution, the distribution of the substances was visualized with ink and observed under a microscope. A fluid containing the lipid mixture was visualized using 6% ink in ethanol, and the fluid containing a hydrophilic substance was visualized using physiological saline, and the results are shown in FIG. 12. As shown in FIG. 12, it can be seen that the lipid mixture and the aqueous solution are efficiently mixed by the microvortex generated when the lipid mixture flows through the micropost structure.

**[Example 6]**

**Optimization of device structural design**

In order to optimize the mixing efficiency according to the size of the designed channels and microposts within the device, the mixing efficiency was confirmed under various conditions including: 1) the initial spacing of microposts; 2) the spacing between microposts; 3) the longitudinal length of microposts; and 4) the flow blockage ratio within the structure of the device.

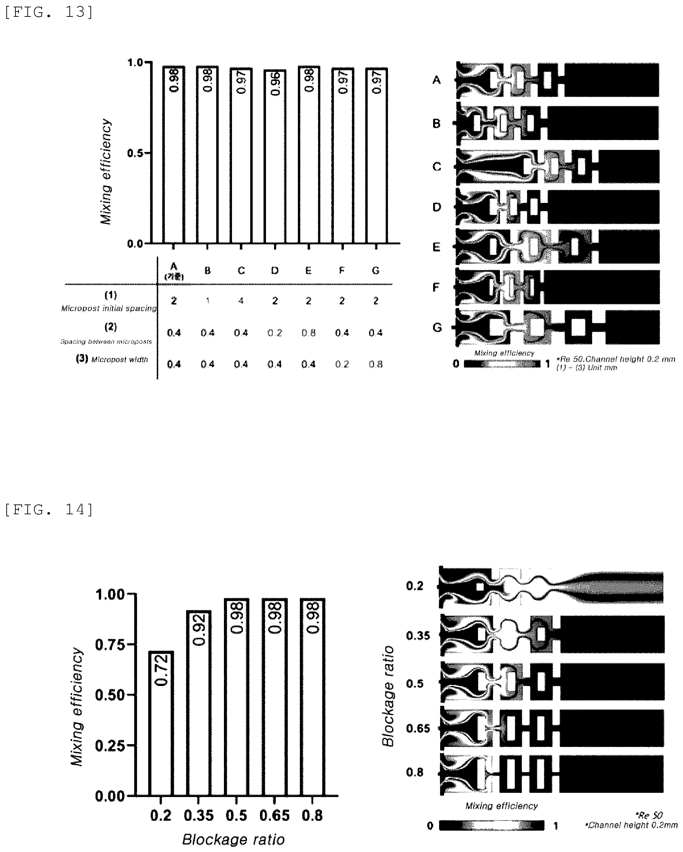

The mixing efficiency for each variable was confirmed under the conditions of a fixed channel height of 0.2 mm, an injection ratio of 1:5.5, and a Reynolds number of 50, and the results are shown in FIGS. 13 and 14.

[TABLE 9]

| Condition | Micropost initial spacing (mm) | Spacing between microposts (mm) | Micropost longitudinal length (mm) | Mixing efficiency |
|---|---|---|---|---|
| A (reference) | 2 | 0.4 | 0.4 | 0.98 |
| B | 1 | 0.4 | 0.4 | 0.98 |
| C | 4 | 0.4 | 0.4 | 0.97 |
| D | 2 | 0.2 | 0.4 | 0.96 |
| E | 2 | 0.8 | 0.4 | 0.96 |
| F | 2 | 0.4 | 0.2 | 0.97 |
| G | 2 | 0.4 | 0.8 | 0.97 |

As shown in FIG. 13 and Table 9 above, it was confirmed that the flow pattern of the fluid passing through the microposts designed in the channel did not change significantly regardless of the spacing and longitudinal length of the microposts, and that a high mixing efficiency (>0.95) was maintained under all conditions.

[TABLE 10]

| Flow blockage ratio | Mixing efficiency |
|---|---|
| 0.2 | 0.72 |
| 0.35 | 0.92 |
| 0.5 | 0.98 |
| 0.65 | 0.96 |
| 0.8 | 0.98 |

On the other hand, as shown in FIG. 14 and Table 10 above, the mixing efficiency increases as the flow blockage ratio (horizontal spacing ratio of the microposts in the channel) increases, and it was confirmed that the mixing efficiency remained consistently at 0.98 or more when the blockage ratio was 0.5.

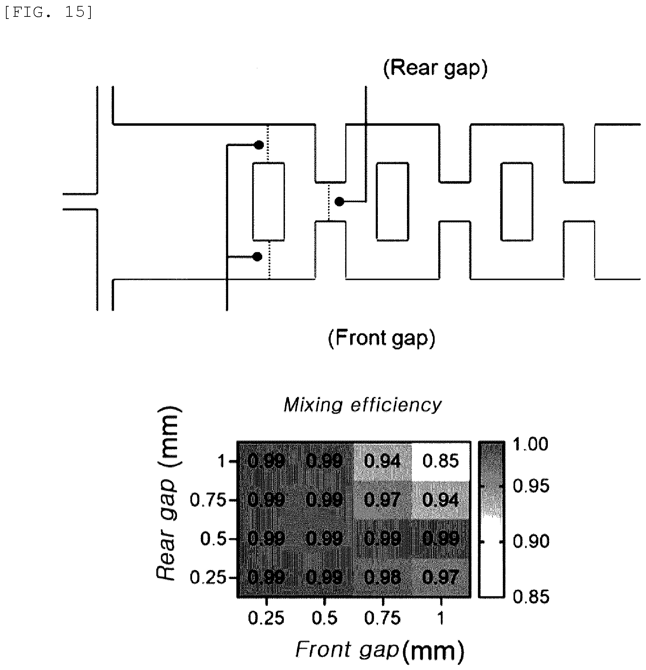

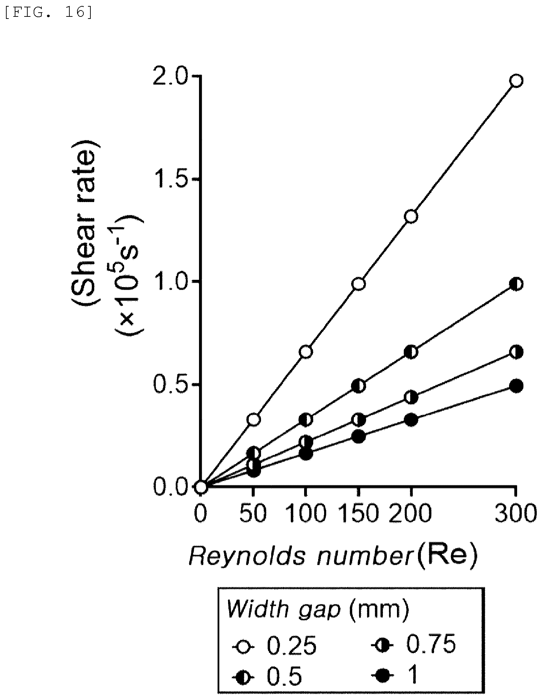

Further, the mixing efficiency according to the front and rear width gap through which the fluid passes is shown in FIG. 15. The shear rate when the front and rear width gaps are the same is shown in FIG. 16. The narrower the width gap through which the fluid passes, the higher the shear rate. Since the shear rate is proportional to the shear stress, it can be said that the shear stress is higher.

That is, when the flow blockage ratio increases, the width of the channel also narrows, and accordingly, the shear stress increases proportionally. As calculated above, high shear stress may cause aggregation and deformation of organic substances present in the fluid when it exceeds a certain value (Bekard, I., et al. The Effects of Shear Flow on Protein Structure and Function. Biopolymers, 95(11), 733-745 (2011)). When considering that the shear stress increases by 1000 dyne/cm$^2$ or more when the flow rate is increased to Re = 300 under the condition of the blockage ratio exceeding 0.65, it can be seen that the flow blockage ratio of the channel may be 0.2 to 0.8, and preferably, when it is in the range of 0.35 to 0.65, it can be seen that high mixing efficiency is not significantly affected by the shear stress.

**[Example 7]**

**Device design for mass production of lipid nanoparticles**

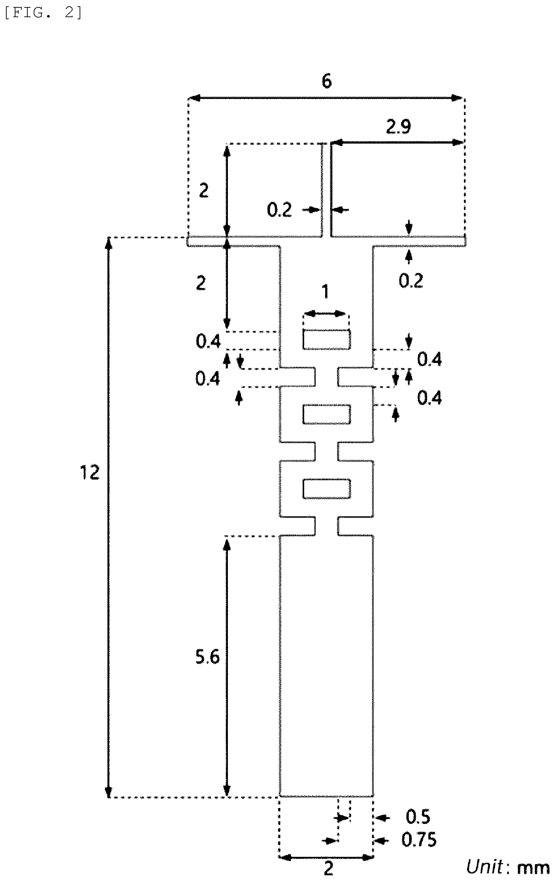

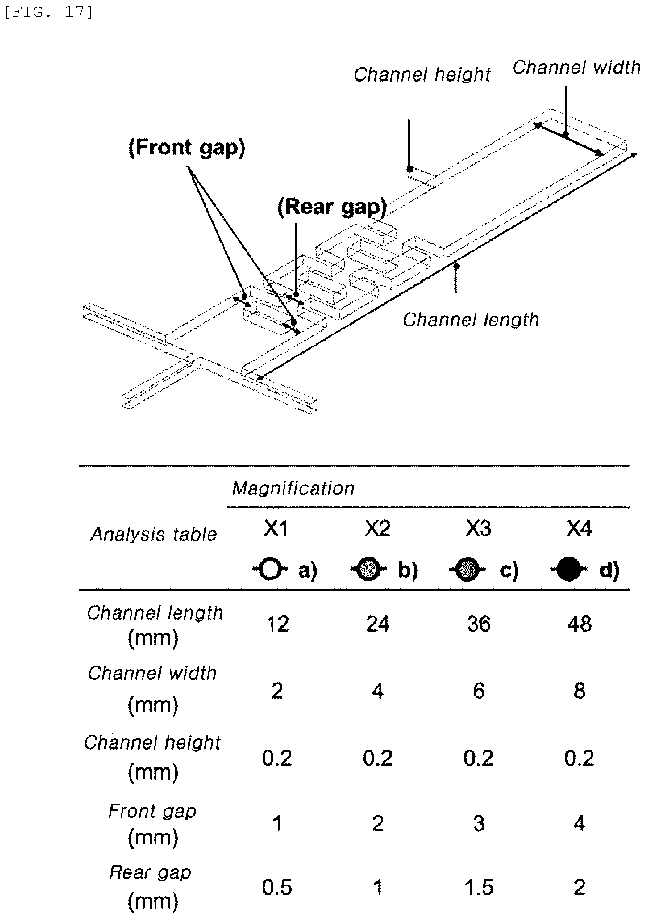

In the present invention, a device for mass production of nanoparticles, including LNPs, was designed. As shown in FIG. 17, when the microfluidic device of FIGS. 1 and 2 was used as a reference (X1), the mixing efficiency, flow rate, and shear rate according to the Reynolds number were analyzed by enlarging the two-dimensional size of the device, i.e., the initial spacing of the microposts, the spacing between the microposts, the longitudinal length of the microposts, and the width gaps between the front and rear sides through which the fluid flows, by two times (X2), three times (X3), and four times (X4), using fluid dynamics techniques. During enlargement, the channel height was fixed at 200 $\mu$m and the flow blockage ratio was maintained at 0.5.

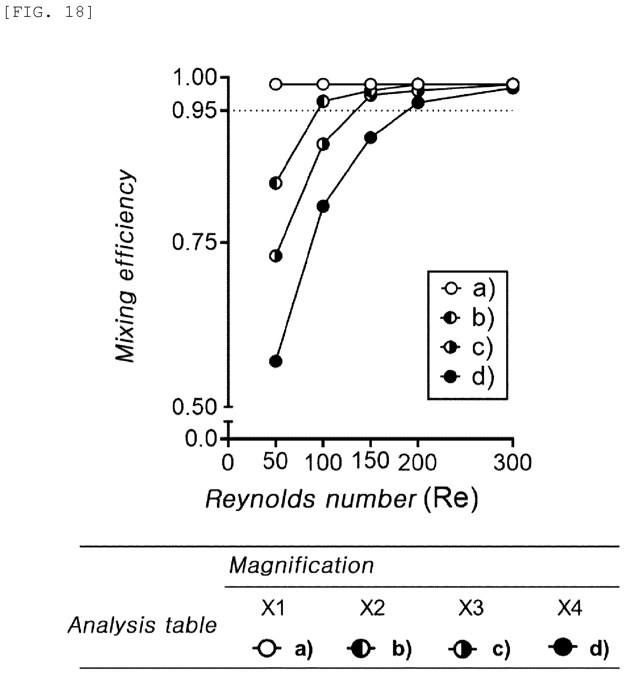

As shown in FIG. 18, it was confirmed that the reference microfluidic device of FIG. 2 exhibited a mixing efficiency of 0.95 or more when the Reynolds number was between 50 and 300. When the device size was doubled (X2), the mixing efficiency decreased to 0.84 at a Reynolds number of 50. However, when the Reynolds number was 100 or more, the mixing efficiency remained at 0.95 or more up to a Reynolds number of 300. When the device size was tripled (X3), the mixing efficiency was 0.95 or more at Reynolds numbers between 150 and 300. When the device size was quadrupled (X4), the mixing efficiency was 0.95 or more at Reynolds numbers between 200 and 300.

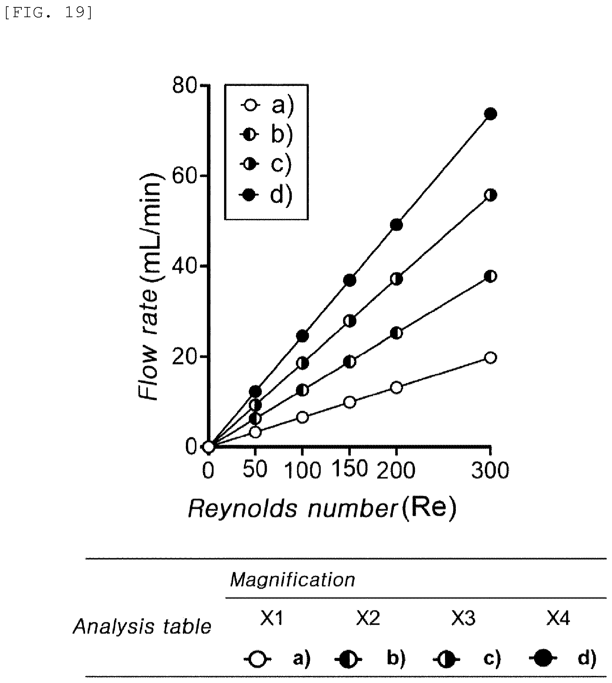

As shown in FIG. 19, it was observed that as the device size increased, only the flow rate needed to be increased to achieve the same Reynolds number. Since an increased flow rate corresponds to an increase in the amount of nanoparticles synthesized per unit time, this confirmed that mass production of nanoparticles is possible through two-dimensional enlargement of the microfluidic device of FIGS. 1 and 2.

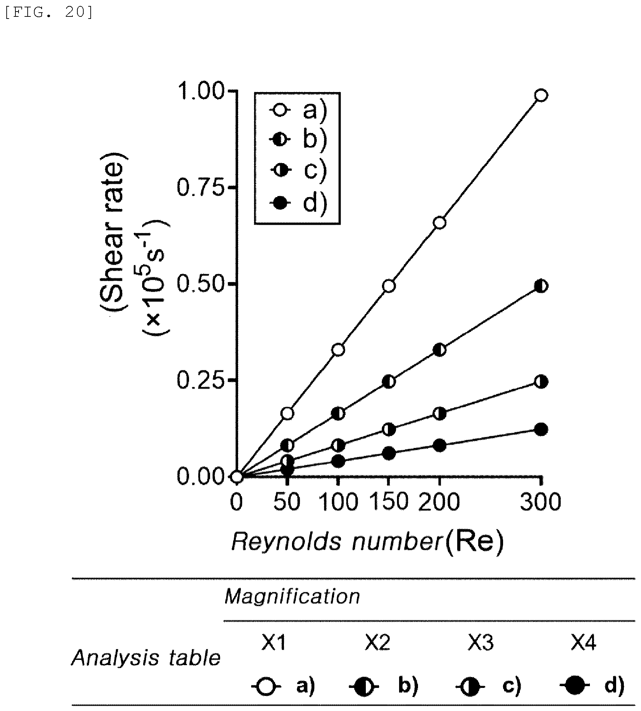

As shown in FIG. 20, it was observed that the shear rate decreased as the device size increased. Since shear rate is proportional to shear stress, it can be concluded that shear stress also decreased as the device size increased. Therefore, enlarging the device does not induce aggregation or deformation of organic substances (such as nucleic acids in the case of LNPs) present in the fluid.

Accordingly, the microfluidic device of the present invention enables mass production of LNPs containing nucleic acids through two-dimensional enlargement of the device size, while preventing aggregation or deformation of organic substances present in the fluid.

**[Example 8]**

**Optimization of the synthesis of lipid nanoparticles**

In the present invention, lipid nanoparticles (LNPs) were synthesized using a microfluidic device as shown in FIG. 1. A lipid mixture was injected into the central inlet channel among the three inlet channels, and an aqueous solution was injected into the two inlet channels on both sides. The lipid mixture and the aqueous solution were efficiently mixed by the microposts inside the device, and the synthesized LNPs were finally obtained through the outlet channel of the micro-mixing channel device.

Ionizable lipids, helper lipids, PEGylated lipids, and sterols may be used as the lipid mixture for LNP synthesis. Specifically, in the present invention, SM-102 (9-heptadecanyl 8-{(2-hydroxyethyl)[6-oxo-6-(undecyloxy)hexyl]amino} octanoate), DSPC (distearoylphosphatidylcholine), POPC (palmitoyloleoylphosphatidylcholine; 1-palmitoyl-2-oleoyl-sn-glycero-3-phosphocholine), DMG-PEG2k (1,2-dimyristoyl-rac-glycero-3-methoxypolyethylene glycol-2000), and cholesterol were used.

Further, a buffer solution of pH 3.0 to 6.0 was required as the aqueous solution for LNP synthesis, and in the present invention, a citrate buffer of pH 3.0 was used.

**8-1 [Synthesis of lipid nanoparticles according to flow rate]**

The lipid mixture used SM-102, DSPC/POPC, cholesterol, and DMG-PEG2k, and the composition ratios were set to 50:10:38.5:1.5, 28:5.7:65.5:0.8, and 20:4:75.5:0.5 (molar ratio), respectively. LNP synthesis was performed while changing the Reynolds number to 12.5, 25, 50, 100 and 200 to compare the sizes of LNPs according to the flow rate. The three ratios of the lipid mixture were set such that the molar ratio of cholesterol in the lipid mixture was increased based on the commonly used ratio of 50:10:38.5:1.5.

With regard to the synthesized LNPs, their uniformity was determined by hydrodynamic size and polydispersity index (PDI) based on the number-weighted size distribution using a Zetasizer Pro Blue instrument with dynamic light scattering (DLS).

[TABLE 11]

| Reynolds number | LNP average size (nm) | PDI |
|---|---|---|
| 12.5 | 83 | 0.102 |
| 25 | 66 | 0.043 |
| 50 | 64 | 0.039 |
| 100 | 67 | 0.066 |
| 200 | 58 | 0.065 |

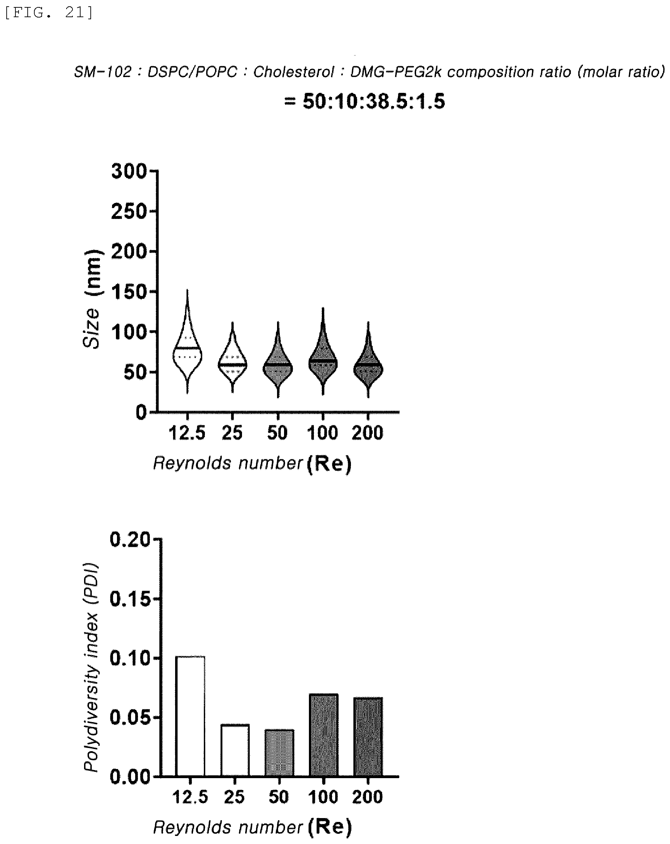

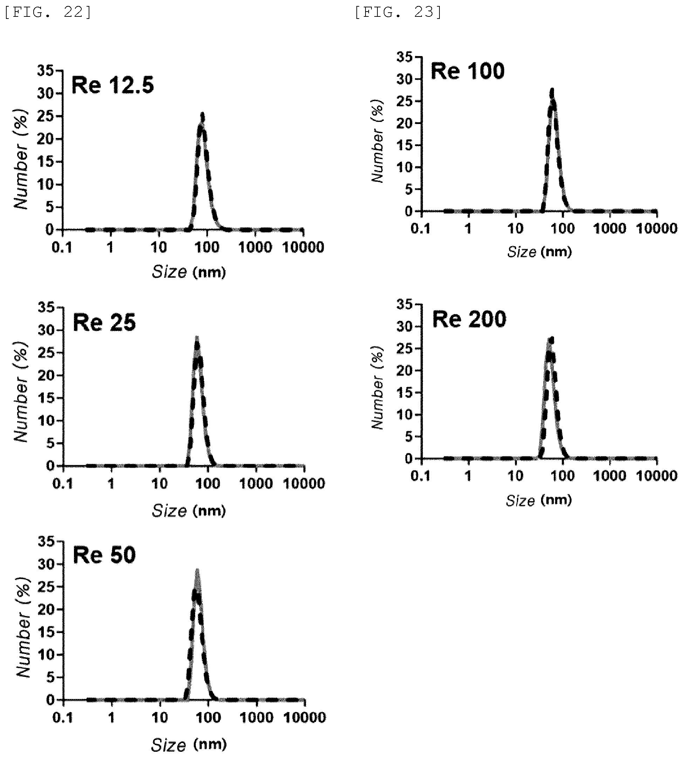

LNPs were synthesized by fixing the molar ratio of SM-102:DSPC/POPC:Cholesterol:DMG-PEG2k at 50:10:38.5:1.5 and altering the Reynolds number to 12.5, 25, 50, 100 and 200. As shown in Table 11 and FIGS. 21 to 23, the sizes of the LNPs were 83, 66, 64, 67 and 58 nm, respectively, and the polydispersity indices (PDIs) of all LNPs were measured to be 0.15 or less, indicating that highly uniform LNPs were synthesized overall. The PDI value considered indicative of stable LNPs may vary depending on the circumstances, but according to the FDA guideline, the PDI of LNPs used as drugs is recommended to be 0.3 or less.

[TABLE 12]

| Reynolds number | LNP average size (nm) | PDI |
|---|---|---|
| 12.5 | 95 | 0.056 |
| 25 | 94 | 0.034 |
| 50 | 102 | 0.028 |
| 100 | 84 | 0.049 |
| 200 | 95 | 0.050 |

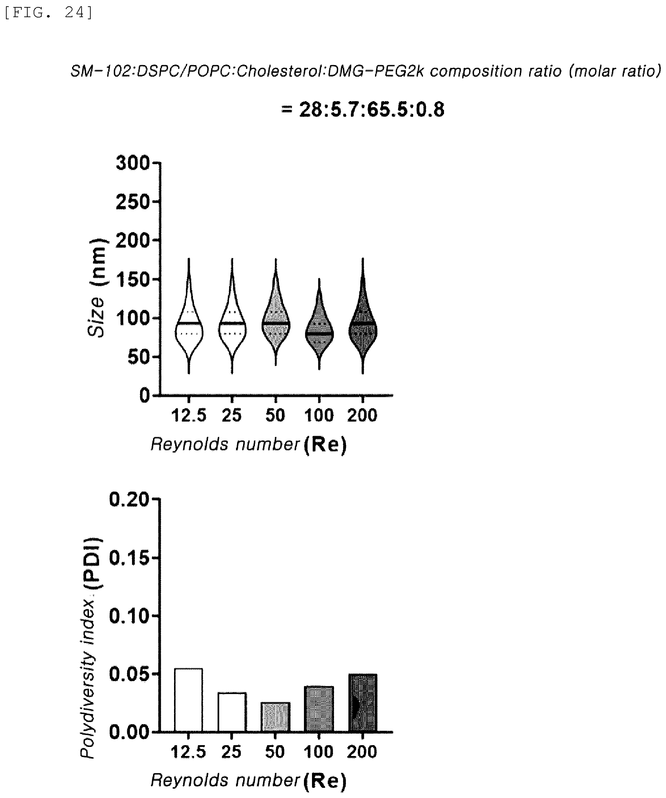

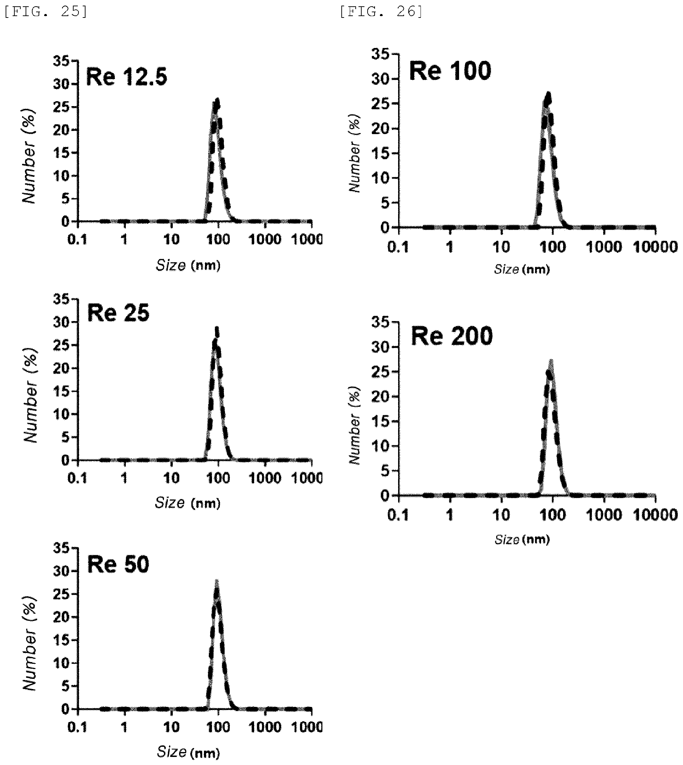

LNPs were synthesized by fixing the molar ratio of SM-102:DSPC/POPC:Cholesterol:DMG-PEG2k at 28:5.7:65.5:0.8 (a condition where only the cholesterol ratio was increased compared to that in Table 1) and altering the Reynolds number to 12.5, 25, 50, 100 and 200. As shown in Table 12 and FIGS. 24 to 26, the sizes of the LNPs were 95, 94, 102, 84 and 95 nm, respectively, and the PDI of all LNPs was measured to be 0.1 or less, indicating that highly uniform LNPs were synthesized.

[TABLE 13]

| Reynolds number | LNP average size (nm) | PDI |
|---|---|---|
| 12.5 | 94 | 0.062 |
| 25 | 83 | 0.068 |
| 50 | 93 | 0.050 |
| 100 | 76 | 0.079 |
| 200 | 86 | 0.062 |

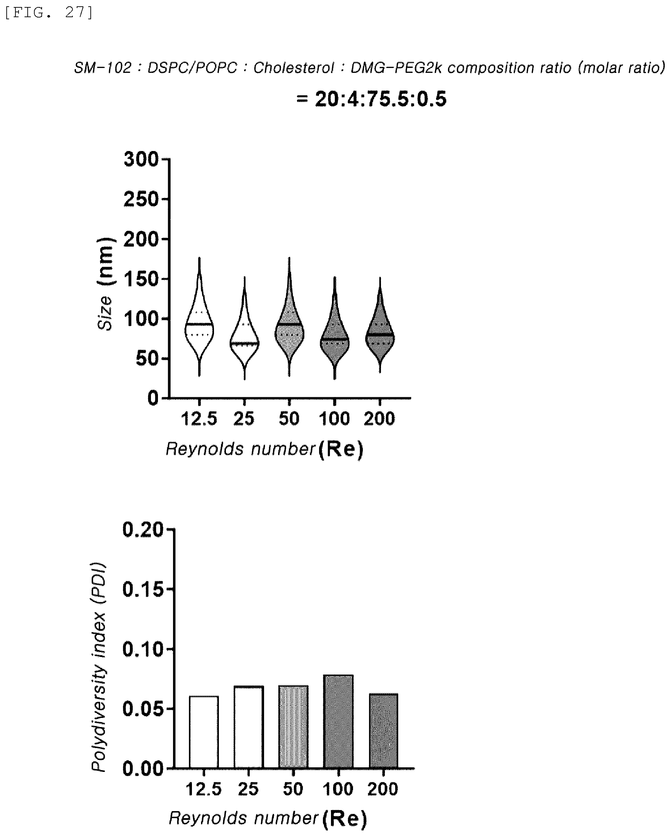

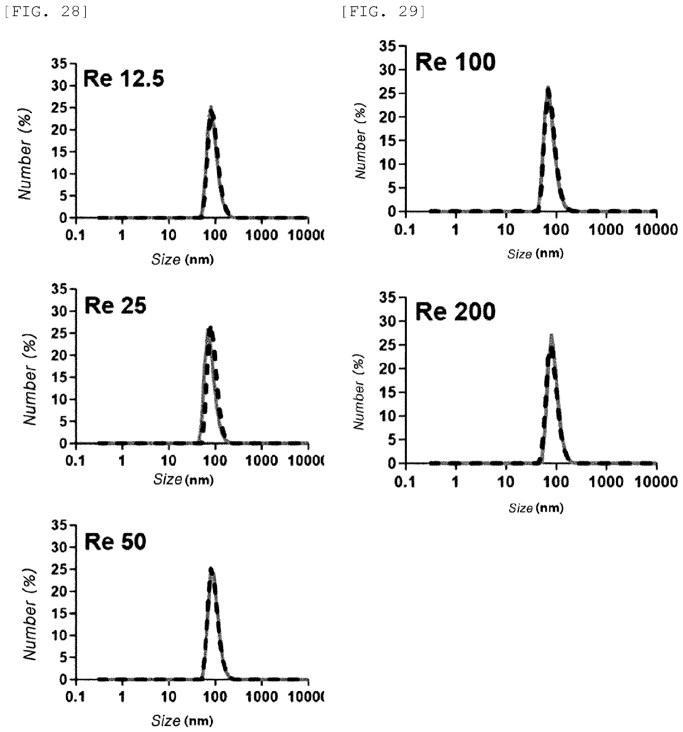

LNPs were synthesized by fixing the molar ratio of SM-102:DSPC/POPC:Cholesterol:DMG-PEG2k at 20:4:75.5:0.5 (a condition where only the cholesterol ratio was increased compared to that in Table 12) and altering the Reynolds number to 12.5, 25, 50, 100 and 200. As shown in Table 13 and FIGS. 27 to 29, the sizes of the LNPs were 94, 83, 93, 76 and 86 nm, respectively, and the PDI of all LNPs was measured to be 0.1 or less, indicating that highly uniform LNPs were synthesized.

That is, when LNPs were synthesized using the microfluidic device shown in FIG. 1, it was confirmed that highly uniform LNPs were synthesized (with PDI values not exceeding 0.1, except for the Reynolds number of 12.5 in Table 11) regardless of the flow rate at various lipid composition ratios. However, it was also confirmed that the LNP size did not show any significant change depending on the flow rate.

**8-2 [Synthesis of lipid nanoparticles according to lipid mixture composition ratio]**

To change the composition ratio of the lipid mixture consisting of SM-102, DSPC/POPC, cholesterol, and DMG-PEG2k, LNPs were synthesized with cholesterol molar ratios in the lipid mixture of 38.5, 55.5, 65.5, 75.5, 86.0 and 98.45, respectively. At this time, the composition ratios of the other lipids (ionizable lipid, helper lipid, and PEGylated lipid), excluding cholesterol, were kept constant.

[TABLE 14]

| Lipid mixture composition ratio (molar ratio) | | | |
|---|---|---|---|
| Ionizable lipid | Helper lipid | Steroid/sterol | PEGylated lipid |
| SM-102 | DSPC/POPC | Chol. | DMG-PEG2k |
| 50 | 10 | 38.5 | 1.5 |
| 36 | 7.5 | 55.5 | 1 |
| 28 | 5.7 | 65.5 | 0.8 |
| 20 | 4 | 75.5 | 0.5 |
| 11 | 2.7 | 86.0 | 0.3 |
| 1 | 0.5 | 98.45 | 0.05 |

The composition ratio of the entire lipid mixture according to the change in the cholesterol molar ratio is shown in Table 14 above. Using the composition ratio of each lipid mixture, LNPs were synthesized using the microfluidic device as in FIG. 1. Based on the results of Example 8-1, the Reynolds number was fixed at 50, at which the smallest PDI and thus the most uniform LNPs were obtained.

[TABLE 15]

| Cholesterol ratio among lipid mixture | LNP average size (nm) | PDI |
|---|---|---|
| 38.5 | 64 | 0.039 |
| 55.5 | 84 | 0.075 |
| 65.5 | 102 | 0.028 |
| 75.5 | 93 | 0.050 |
| 86.0 | 103 | 0.154 |
| 98.45 | 105 | 0.274 |

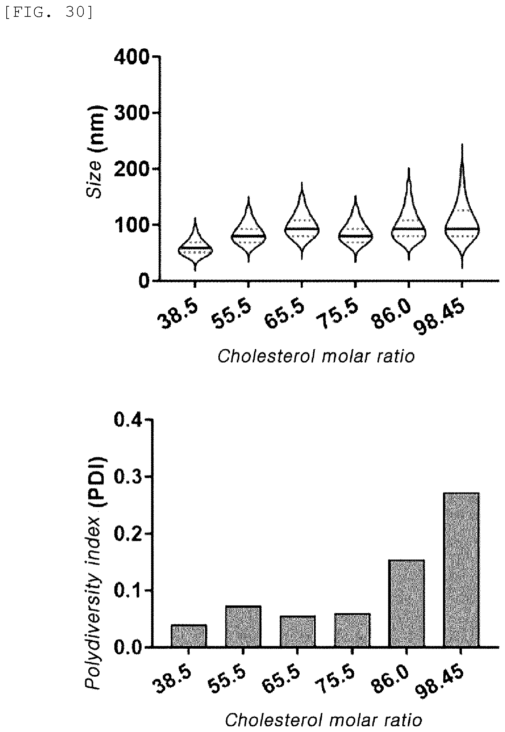

As shown in Table 15 above, the results of LNP size and PDI obtained by adjusting the composition ratio of the lipid mixture are presented in Table 15. As shown in FIG. 30 and Table 15, the sizes of the synthesized LNPs at cholesterol molar ratios in the lipid mixture of 38.5, 55.5, 65.5, 75.5, 86.0 and 98.45 were measured to be 64, 84, 102, 93, 103 and 105 nm, respectively.

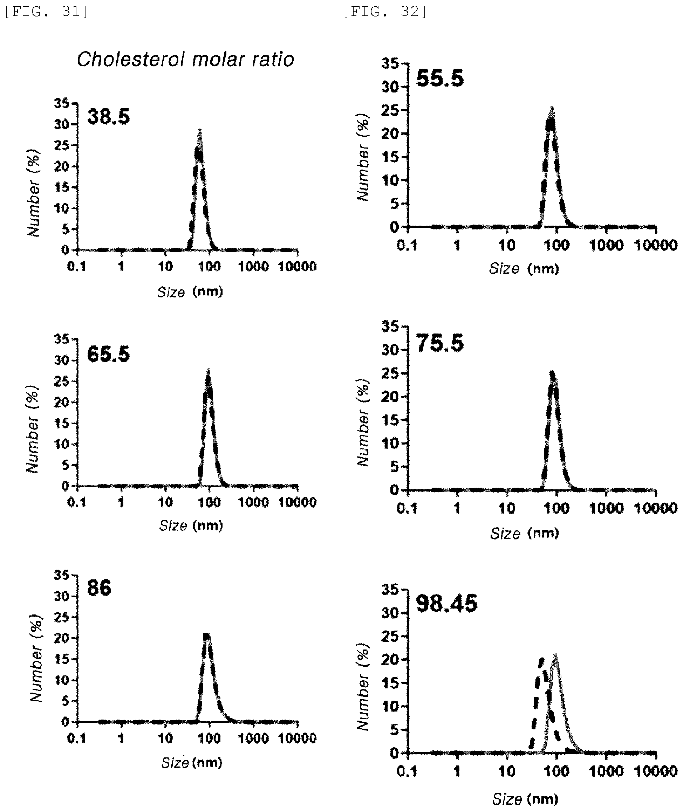

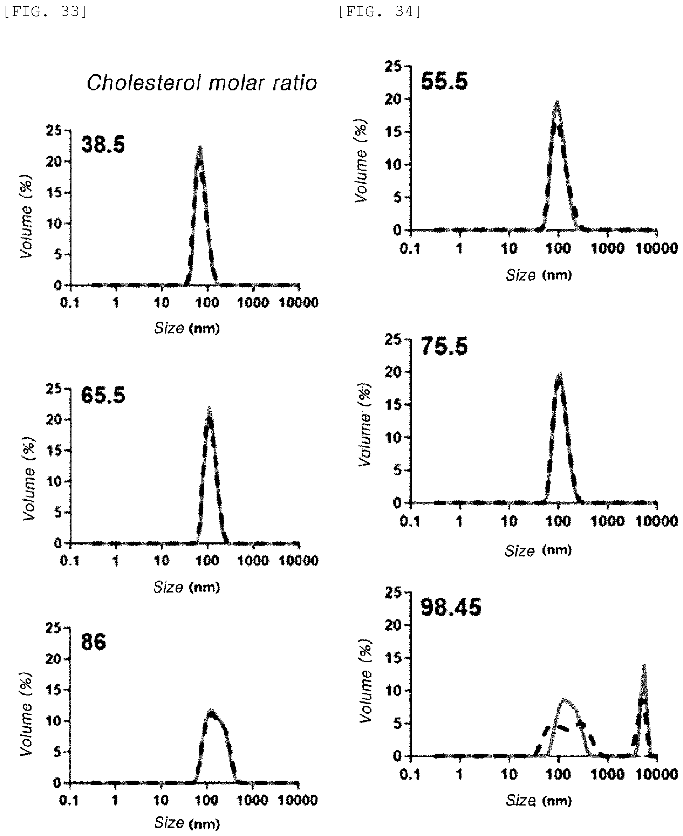

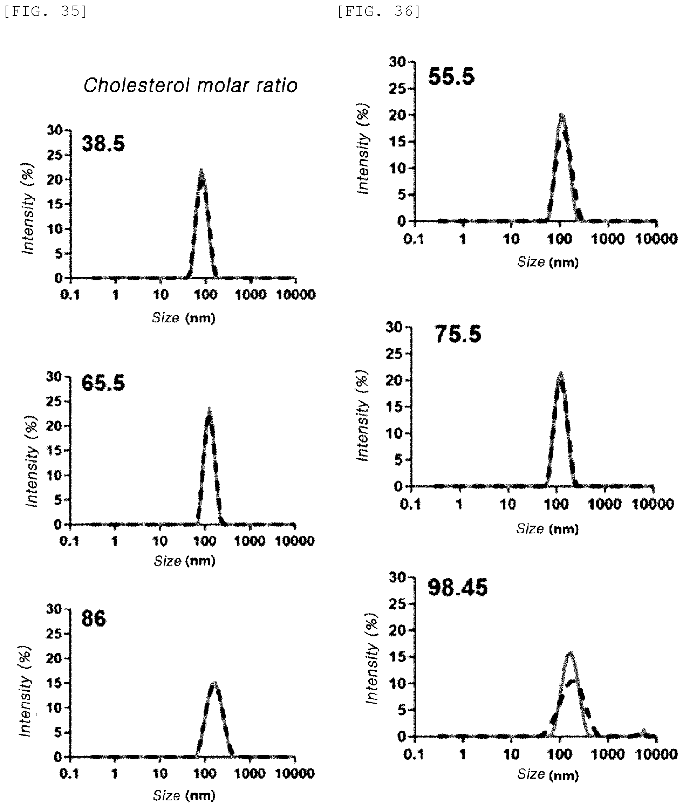

The uniformity of LNPs synthesized through the microfluidic device was confirmed by PDI. Up to a cholesterol molar ratio of 86.0, the PDI was measured to be 0.2 or less, indicating that the LNPs were synthesized with high uniformity. The uniformity of the synthesized LNPs was also confirmed by the narrow single peaks observed in the number-weighted size distribution in FIGS. 31 and 32, the volume-weighted size distribution in FIGS. 33 and 34, and the intensity-weighted size distribution in FIGS. 35 and 36.

When the microfluidic device was used, uniform LNP sizes were obtained even when the cholesterol molar ratio in the lipid mixture was 86.0, corresponding to 80% of the total lipid mixture composition, which is more than twice the generally used ratio of 38.5. Furthermore, when using the microfluidic device, LNPs could also be synthesized under an extreme lipid mixture composition with a cholesterol molar ratio of 98.45. However, in this case, the PDI was measured to be 0.274, indicating reduced monodispersity compared with other conditions. This increase in PDI was attributed to aggregation of LNPs caused by the excessive amount of cholesterol.

Additionally, under conditions where the cholesterol molar ratio was 60 or less, the LNP size tended to increase as the cholesterol ratio increased; however, no further change in LNP size was observed when the cholesterol molar ratio reached 65.5 or higher.

**8-3 [Optimization of the synthesis of size-specific lipid nanoparticles]**

As seen from the results of Examples 8-1 and 8-2 above, the size of the LNPs synthesized using the microfluidic device was in the range of 60 to 100 nm. To synthesize LNPs having a size of 50 nm or less, the molar ratio of PEGylated lipids in the lipid mixture was increased. In this case, the composition ratio of the lipid mixture SM-102:DSPC/POPC:Cholesterol:DMG-PEG2k was set to 48.5:9.5:37.5:4.5. Further, to examine whether the LNP size varied according to the Reynolds number, synthesis was performed at Reynolds numbers of 50 and 100.

[TABLE 16]

| Reynolds number | LNP average size (nm) | PDI |
|---|---|---|
| 50 | 35 | 0.212 |
| 100 | 36 | 0.155 |

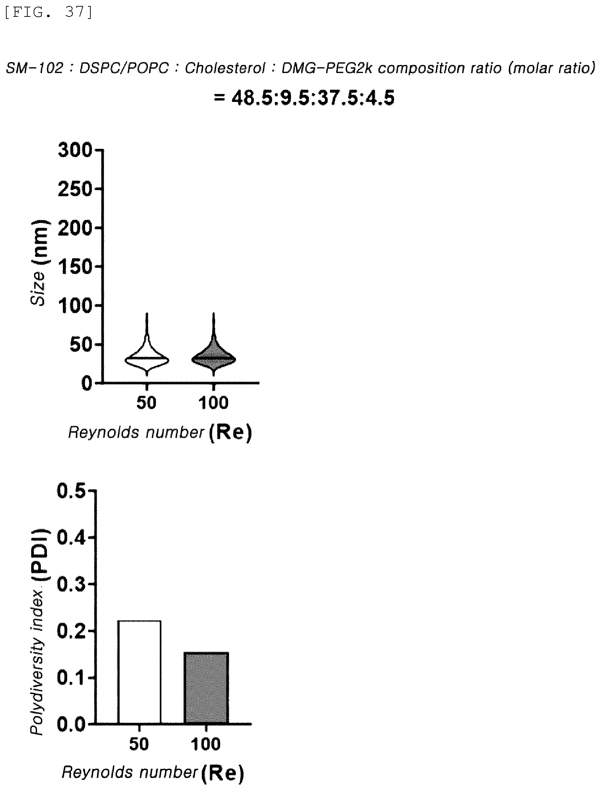

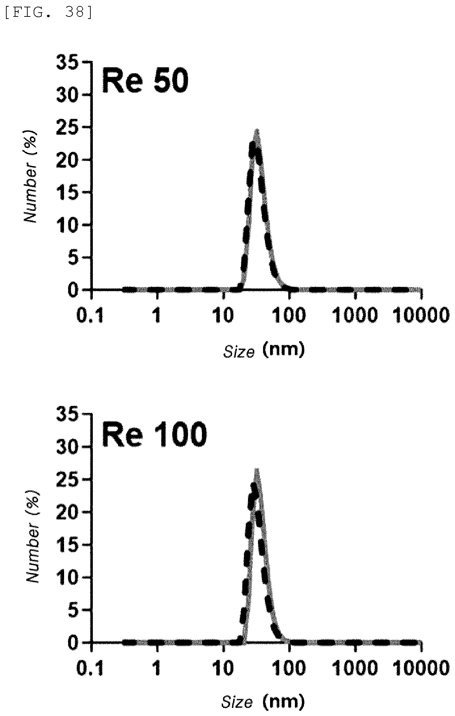

As shown in Table 16 above and FIGS. 37 and 38, when the Reynolds numbers were 50 and 100, the LNPs were synthesized with sizes of 35 nm and 36 nm, respectively, and it was confirmed that they were synthesized with a relatively uniform PDI of about 0.2.

[TABLE 17]

| Target LNP size (nm) | Reynolds number | Lipid mixture composition ratio (molar ratio) | | | |
|---|---|---|---|---|---|
| | | Ionizable lipid | Helper lipid | Steroid/sterol | PEG-lipid |
| | | SM-102 | DSPC/POPC | Chol. | DMG-PEG2k |
| 30 | 100 | 48.5 | 9.5 | 37.5 | 4.5 |
| 60 | 50 | 50 | 10 | 38.5 | 1.5 |
| 90 | 25 | 28 | 5.7 | 65.5 | 0.8 |

When the results of the finally synthesized LNPs were summarized, Table 17 above shows the Reynolds numbers and lipid mixture composition ratios for synthesizing very uniform LNPs with specific sizes of 30 nm, 60 nm and 90 nm using the microfluidic device illustrated in FIG. 1.

An average diameter of LNPs generally used as mRNA vaccines falls within the range of 80 to 100 nm. LNPs with a smaller size (about 60 nm) are difficult to manufacture because they impose high curvature stress on the particles, the stability of the particles tends to be lower, and the loading efficiency of the particles is poor. On the other hand, as the size of the LNP decreases, the surface area of the particles increases, and the drug delivery efficiency to the target organ tends to improve.

Further, the size of LNPs also affects the targeting characteristics of the particles to each organ of the body. In general, it is known that organs such as the liver, which contain many microvessels, exhibit reduced targeting characteristics as the size of the LNPs increases. Furthermore, research results have revealed that small LNPs less than 60 nm (particularly in the 30 nm range) exhibit excellent targeting characteristics for dendritic cells in lymph nodes and various other immune cells.

For the above reasons, it is very important to set an appropriate particle size according to the intended use of LNPs and to establish a preparation method for implementing the same. The present invention provides a method for the production of LNPs with a size suitable for the intended use by regulating the Reynolds number and the composition ratio of particles using the microfluidic device.

**[Example 9]**

**Optimization of the synthesis of size-specific lipid nanoparticles encapsulating mRNA**

Lipid nanoparticles (mRNA LNPs) encapsulating messenger ribonucleic acid (mRNA) were synthesized using the same microfluidic device used in Example 8. The synthesis was carried out in the same manner as in Example 8, and mRNA was prepared in a citrate buffer solution at pH 3.0 such that the aqueous solution contained mRNA at an N/P ratio of 6.

mRNA LNPs were synthesized by applying the size-based LNP synthesis conditions set forth in Table 17 of Example 8-3 above. At this time, the mRNAs used were mCherry mRNA with a length of 996 nucleotides and Cas9 mRNA with a length of 4521 nucleotides, and mCherry LNPs and Cas9 LNPs were synthesized in different sizes using two types of mRNAs with different lengths.

[TABLE 18]

| Target LNP size (nm) | Reynolds number | Lipid mixture composition ratio (molar ratio) SM-102: DSPC/POPC; Chol.:DMG-PEG2k | mCherry LNP average size (nm) | PDI | Encapsulation efficiency (%) | Loading efficiency (%) |
|---|---|---|---|---|---|---|
| 30 | 100 | 48.5:9.5:37.5:4.5 | 40 | 0.105 | 84 | 38 |
| 60 | 50 | 50:10:38.5:1.5 | 57 | 0.054 | 95 | 39 |
| 90 | 25 | 28:5.7:65.5:0.8 | 89 | 0.052 | 100 | 44 |

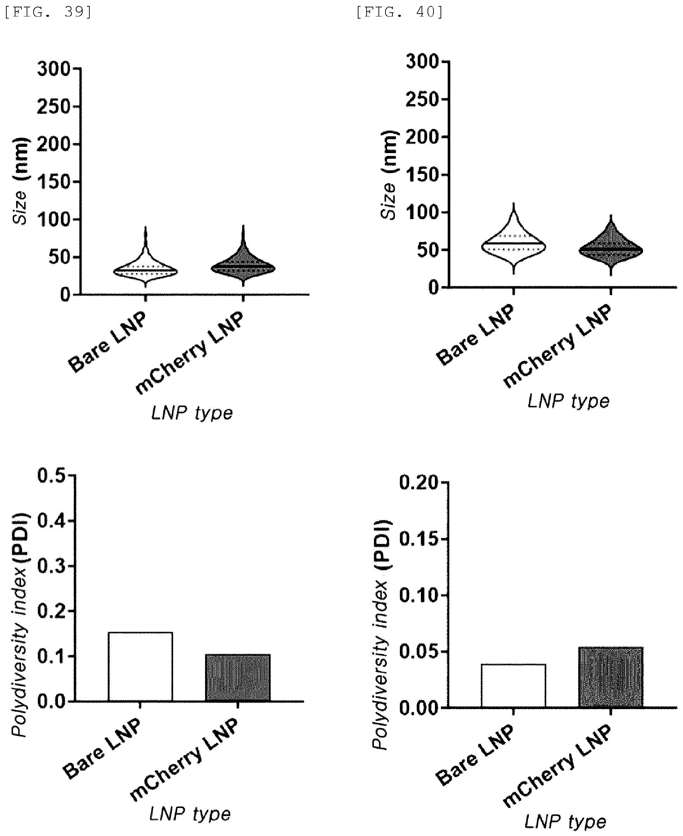

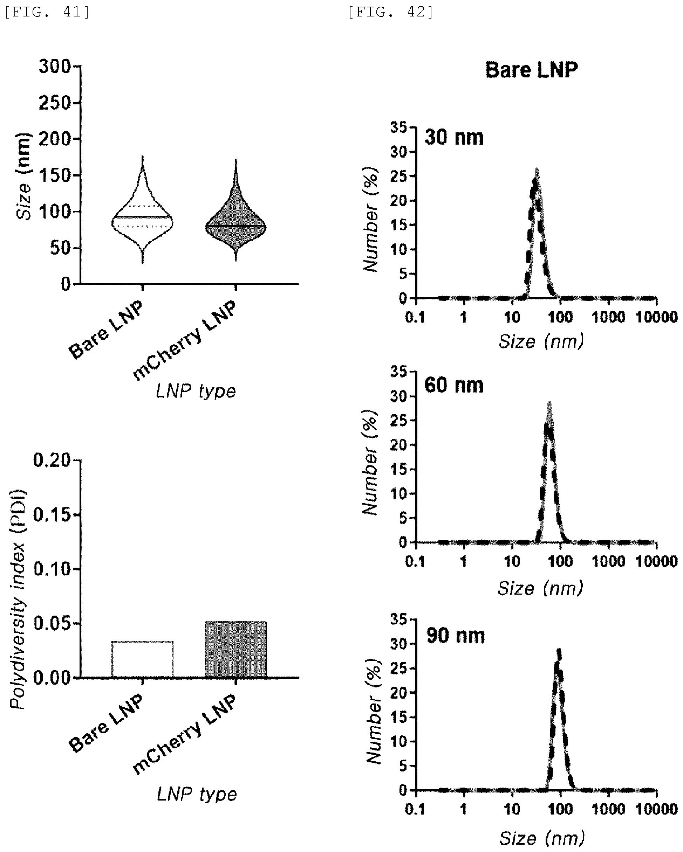

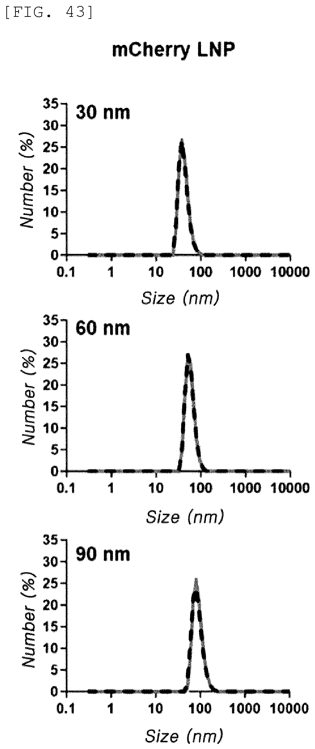

As shown in Table 18 and FIGS. 39, 40 and 41, the sizes of mCherry LNPs synthesized targeting 30, 60 and 90 nm were measured to be 40, 57 and 89 nm, respectively, and the PDIs at this time were 0.105, 0.054 and 0.052, respectively, indicating that very uniform LNPs were synthesized. Further, the similarity in size distribution between mCherry LNPs and Bare LNPs (without mRNA) was also confirmed. The uniformity of mCherry LNPs by size was also confirmed through the number-weighted size distribution results in FIGS. 42 and 43.

[TABLE 19]

| Target LNP size (nm) | Reynolds number | Lipid mixture composition ratio (molar ratio) SM-102: DSPC/POPC; Chol.:DMG-PEG2k | Cas9 LNP average size (nm) | PDI | Encapsulation efficiency (%) | Loading efficiency (%) |
|---|---|---|---|---|---|---|
| 30 | 100 | 48.5:9.5:37.5:4.5 | 37 | 0.190 | 74 | 44 |
| 60 | 50 | 50:10:38.5:1.5 | 57 | 0.036 | 98 | 29 |
| 90 | 25 | 28:5.7:65.5:0.8 | 90 | 0.014 | 100 | 37 |

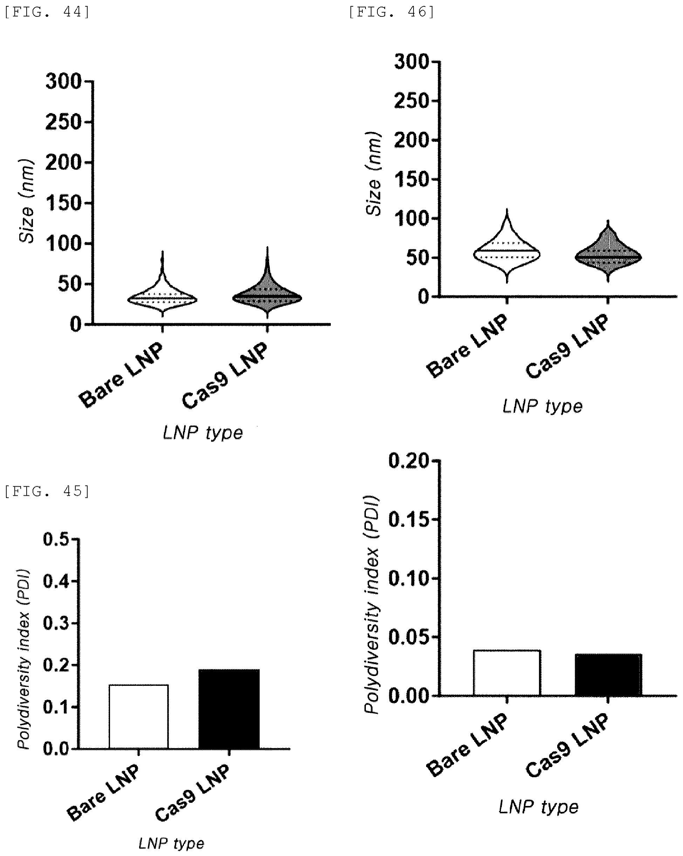

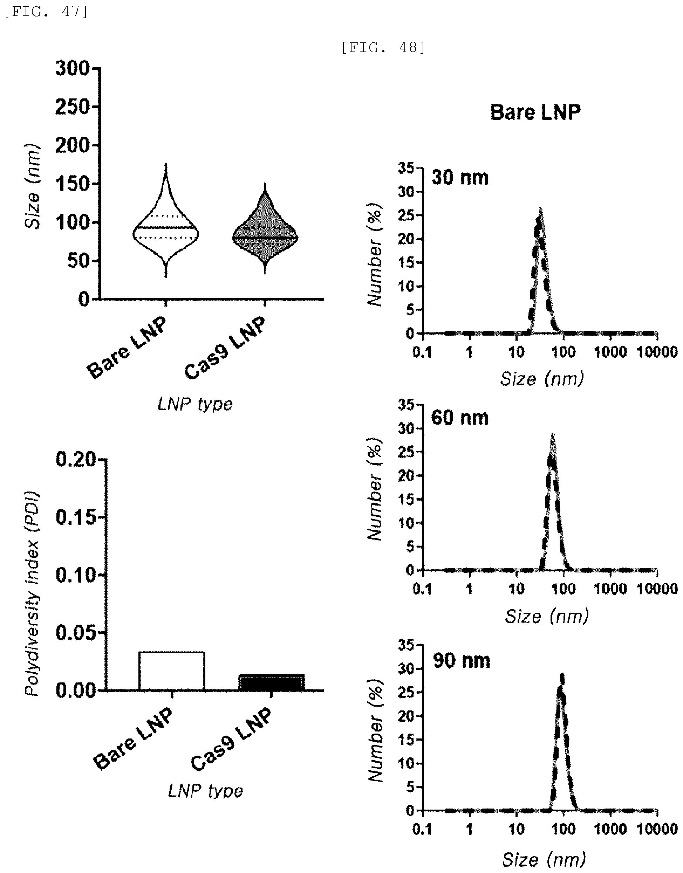

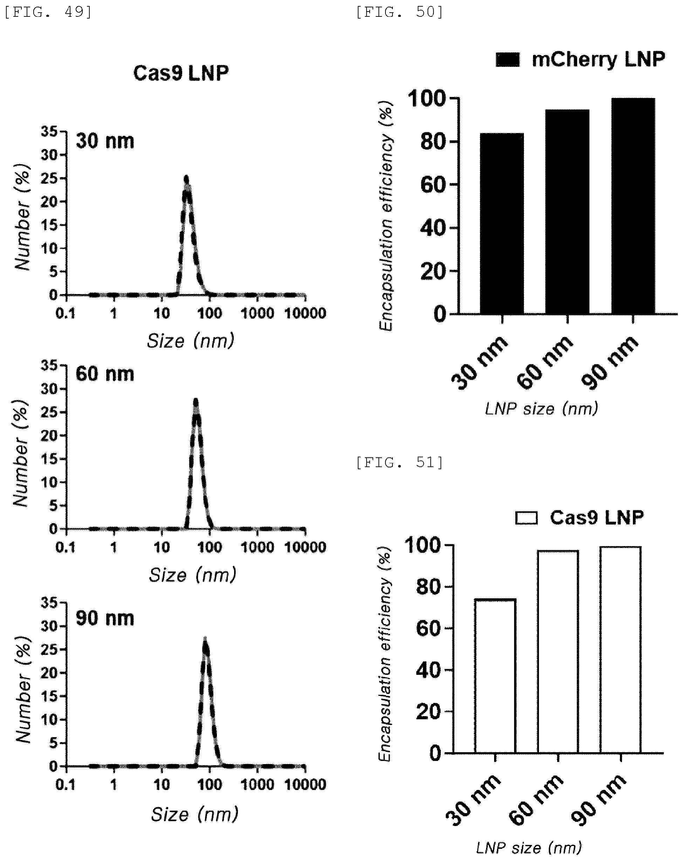

As shown in Table 19 and FIGS. 44, 45, 46 and 47, the sizes of mCherry LNPs synthesized targeting 30, 60 and 90 nm were measured to be 37, 57 and 90 nm, respectively, and the PDI at this time was 0.190, 0.036 and 0.014, respectively. It can be seen that the Cas9 LNPs targeting 60 nm and 90 nm were synthesized very uniformly, and the Cas9 LNP targeting 30 nm also has a PDI of 0.2 or less, indicating that the uniformity of the nanoparticles is still secured. Further, the similarity in the size distributions of Cas9 LNP and Bare LNP could also be confirmed. The uniformity of Cas9 LNP by size could also be confirmed through the number-weighted size distribution results in FIGS. 48 and 49.

The nucleic acid encapsulation efficiency (EE) is calculated from each mRNA LNP based on fluorescence intensity, by detecting the total mRNA and the unencapsulated mRNA (free mRNA). The value is calculated according to

the following equation: $\text{Encapsulation Efficiency (\%)} = \frac{\text{Total mRNA} - \text{Free mRNA}}{\text{Total mRNA}} \times 100$. Similarly, the nucleic acid loading efficiency (LE) is calculated from each mRNA LNP based on fluorescence intensity, by detecting the total mRNA and the unencapsulated mRNA (free mRNA). The value is calculated according to the following equation:

$\text{Loading Efficiency (\%)} = \frac{\text{Total mRNA} - \text{Free mRNA}}{\text{Feeding mRNA}} \times 100$. The difference between the nucleic acid encapsulation efficiency and the nucleic acid loading efficiency lies in the denominator, i.e., whether the denominator is the detected total mRNA or the feeding mRNA.

That is, in summary, the nucleic acid encapsulation efficiency (EE) is an indicator of the quality of the final product, reflecting the mRNA content, whereas the nucleic acid loading efficiency (LE) is an indicator of the efficiency (or yield) with which the input mRNA is incorporated into the lipid nanoparticles (LNPs).

As shown in Table 18 and FIG. 50, the nucleic acid encapsulation efficiencies of mCherry LNPs with sizes of 30, 60, and 90 nm were measured to be 84%, 95%, and 100%, respectively, and as shown in Table 19 and FIG. 51, the nucleic acid encapsulation efficiencies of Cas9 LNPs with sizes of 30, 60, and 90 nm were 74%, 98%, and 100%, respectively. Regardless of the type of mRNA, mRNA LNPs with sizes of 60 nm or 90 nm achieved a nucleic acid encapsulation efficiency of 95% or more, indicating that the lipid mixture and the mRNA were mixed very efficiently by the microfluidic device. Even in the case of 30-nm mRNA LNPs, where the space available for nucleic acid encapsulation is relatively limited, the nucleic acid encapsulation efficiencies remained high-84% for mCherry LNPs and 74% for Cas9 LNPs. This also indicates that the lipid mixture and the mRNA were efficiently mixed by the microfluidic device. Such high nucleic acid encapsulation efficiencies suggest that the probability of impurity issues caused by unencapsulated mRNA is very low.

As shown in Table 18, the nucleic acid loading efficiencies of mCherry LNPs with sizes of 30, 60, and 90 nm were 38%, 39%, and 44%, respectively. As shown in Table 19, the nucleic acid loading efficiencies of Cas9 LNPs with sizes of 30, 60, and 90 nm were 44%, 29%, and 37%, respectively. The consistently high nucleic acid loading efficiencies of 30% or more, regardless of the target size or mRNA type, further confirm that the lipid mixture and the mRNA were efficiently mixed by the microfluidic device. In addition, high nucleic acid loading efficiency means that the loss of raw materials is reduced.

Accordingly, the microfluidic device of the present invention enables the synthesis of size-controlled mRNA LNPs with high uniformity, irrespective of the length of the mRNA, and provides an excellent synthesis platform for developing highly efficient LNP-based nucleic acid therapeutics or vaccines that combine stability and functionality for various diseases.

**[Example 10]**

**Optimization of the synthesis of size-specific lipid nanoparticles encapsulating oligonucleotides**

Oligo LNPs encapsulating oligonucleotides were synthesized using the same microfluidic device used in Examples 8 and 9. The synthesis was carried out in accordance with the method of Example 8, using the conditions shown in Table 17 above. The oligonucleotides were prepared in a citrate buffer solution at pH 3.0 such that the aqueous solution contained the oligonucleotides at an N/P ratio of 6.

Each Oligo LNP was synthesized under the size-specific LNP synthesis conditions established in Example 8-3. At this time, the oligonucleotide used was a DNA strand consisting solely of 20 thymine (T) bases (20-mer).

[TABLE 20]

| Target LNP size (nm) | Reynolds number | Lipid mixture composition ratio (molar ratio) SM-102: DSPC/POPC; Chol.:DMG-PEG2k | Oligo LNP average size (nm) | PDI | Encapsulation efficiency (%) | Loading efficiency (%) |
|---|---|---|---|---|---|---|
| 30 | 100 | 48.5:9.5:37.5:4.5 | 50 | 0.029 | 90 | 53 |
| 60 | 50 | 50:10:38.5:1.5 | 99 | 0.053 | 89 | 48 |
| 90 | 25 | 28:5.7:65.5:0.8 | 132 | 0.036 | 91 | 47 |

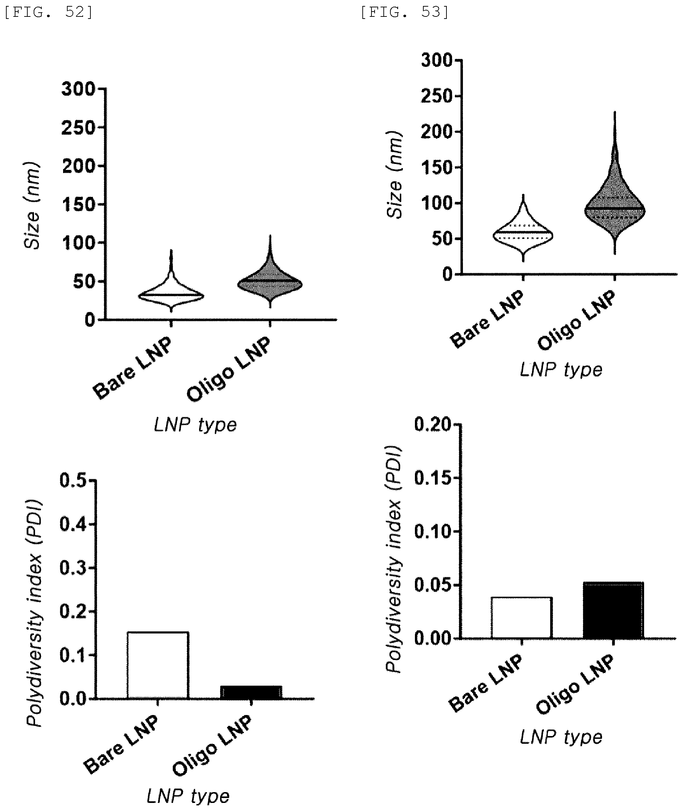

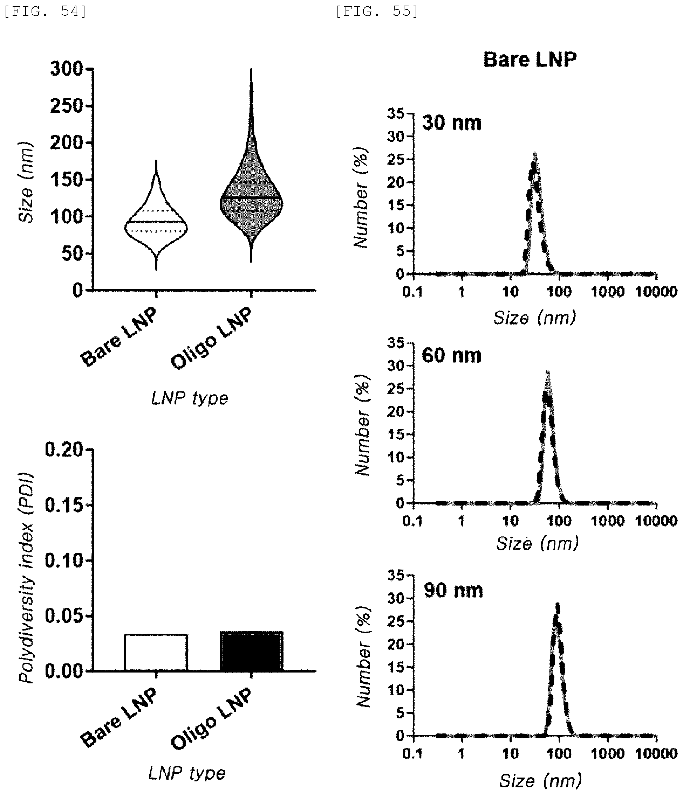

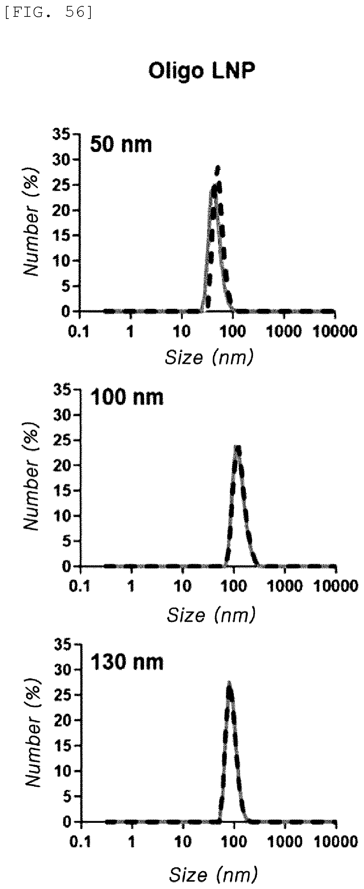

As shown in Table 20 above and FIGS. 52, 53 and 54, the sizes of Oligo LNPs synthesized with target sizes of 30, 60 and 90 nm were measured to be 50, 99 and 132 nm, respectively, indicating that they were synthesized at sizes 20 to 40 nm larger than the targets. The PDIs at this time were 0.029, 0.053 and 0.036, respectively. Although the synthesized sizes were larger than the targets, it was confirmed that the Oligo LNPs were still synthesized with high uniformity upon oligonucleotide encapsulation at all sizes. Further, the uniformity of the Oligo LNPs by size was also confirmed through the number-weighted size distribution results in FIGS. 55 and 56.

Based on the fluorescence intensity of the Oligo LNP, the total oligonucleotide and the free oligonucleotide were detected, and the obtained values were used to calculate the nucleic acid encapsulation efficiency (EE) and the nucleic acid loading efficiency (LE) of the oligonucleotide. The nucleic acid encapsulation efficiency was calculated according to the following equation: $EE\ (\%) = \frac{Total\ oligonucleotide - Free\ oligonucleotide}{Total\ oligonucleotide} \times 100$, while the nucleic acid loading efficiency is calculated by the following equation: $LE\ (\%) = \frac{Total\ oligonucleotide - Free\ oligonucleotide}{Feeding\ oligonucleotide} \times 100$. *Feeding oligonucleotide*

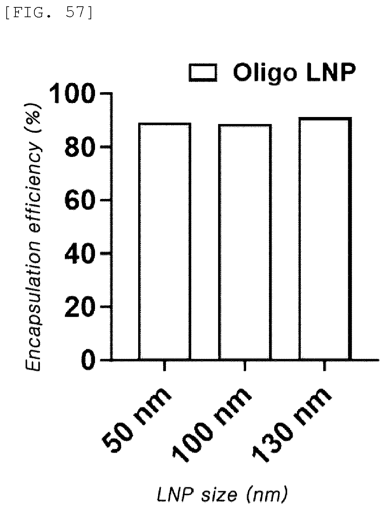

As shown in Table 20 and FIG. 57, the nucleic acid encapsulation efficiencies of the Oligo LNPs of 50, 100 and 130 nm were 90, 89 and 91%, respectively, and the nucleic acid loading efficiencies were 53, 48 and 47%, respectively. Through a nucleic acid encapsulation efficiency of 90%, it was confirmed that the probability of impurity problems caused by unencapsulated oligonucleotides is low, and through a nucleic acid loading efficiency of 45% or more, it was confirmed that raw material loss is low. In other words, the lipid mixture and oligonucleotides were efficiently mixed by the microfluidic device.

Unlike mRNA LNPs, Oligo LNPs being synthesized larger than the target size may be attributed to the difference in the nucleic acids contained therein. Oligonucleotides are composed of 20 nucleotides, whereas mRNAs are composed of 1,000 to 5,000 nucleotides, showing a length difference of 50 to 250 times. When comparing the nucleic acid loading efficiencies of LNPs of target sizes 30, 60 and 90 nm in Tables 18, 19 and 20 above, it was found that Oligo LNPs were higher than mRNALNPs by up to 15%, 20% and 10%, respectively. This result appears to be due to an increase in the amount of short oligonucleotides loaded in the LNPs, leading to an increase in size.

That is, the microfluidic device of the present invention is a device capable of synthesizing Oligo LNPs at a specific size with high uniformity, and may be an excellent synthesis platform for developing highly efficient LNP-based therapeutic agents or vaccines with both stability and functionality for various diseases.

**[Example 11]**

**Confirmation of intracellular protein expression according to size-specific mCherry LNP treatment**

To verify the ability of LNP-based mRNA delivery within cells, the level of protein expression in cells treated with mRNA LNP was evaluated by fluorescence imaging using a confocal microscope. Human glioblastoma U87MG, human hepatoma HepG2, and human monocyte THP-1 cell lines were used to confirm intracellular protein expression of mRNA. mCherry mRNA, which emits fluorescence, was used as a reporter to verify protein expression. mCherry LNPs were synthesized at target sizes of 30, 60 and 90 nm in the same manner as in Example 9.

In human glioblastoma U87MG cells, the expression of mCherry protein depending on the size of mCherry LNP was assessed. U87MG cells were treated with 200 ng of mRNA using mCherry LNPs of each size (30, 60 and 90 nm) and cultured for 24 hours under 5% $CO_2$ at 37 °C. After removing residual mCherry LNPs from the culture medium, the cells were further cultured for 24 hours, and the medium was replaced for protein expression analysis. The nuclei and cytoskeleton of the cells were then stained. After staining, U87MG cells were fixed with 1 mL of 4% paraformaldehyde solution for 15 minutes. Fluorescence images of mCherry protein expressed in each fixed cell were captured using a confocal microscope equipped with a filter having an excitation wavelength of 587 nm and an emission wavelength of 610 nm.

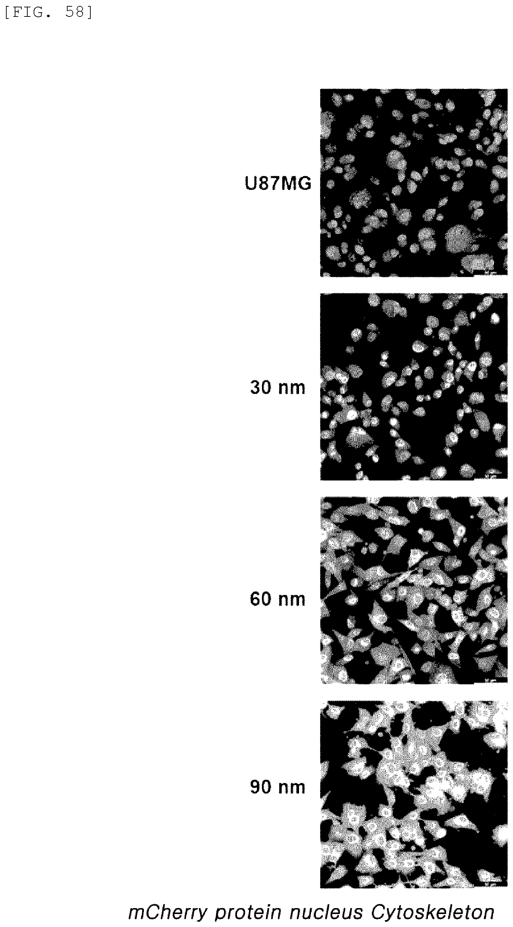

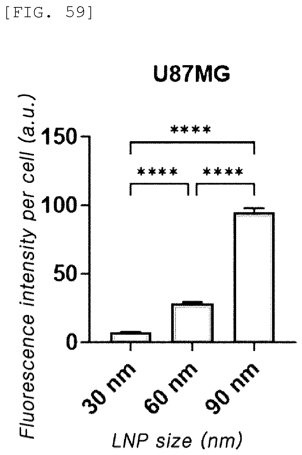

As shown in FIG. 58, the expression of mCherry protein in U87MG cells was visualized by fluorescence imaging. The expression of mCherry protein in the cytoplasmic region was confirmed by the overlap of cytoskeletal fluorescence staining (green) and mCherry fluorescence (red). When U87MG cells were treated with the same amount of 200 ng of mCherry LNPs at three different sizes (30, 60 and 90 nm), mCherry protein expression increased with LNP size. As shown in the quantitative results of FIG. 59, the expression level was approximately 4-fold and 14-fold higher with 60 nm and 90 nm LNPs, respectively, compared with the smallest 30 nm LNP. Furthermore, expression with the largest 90 nm LNP was about 3-fold higher than with the medium-sized 60 nm LNP.

In HepG2 cells, a human hepatoma cell line, the effect of mRNA LNP concentration on protein expression was evaluated, and mCherry LNPs were synthesized at two sizes (60 and 90 nm). HepG2 cells were treated with 20, 200, or 500 ng of each 60 and 90 nm mCherry LNP and then cultured for 24 hours under 5% $CO_2$ and 37 °C. Residual LNPs in the culture medium were removed, and the cells were further cultured for an additional 24 hours. To confirm mCherry protein expression, the culture medium was replaced, and the nuclei and cytoskeleton were stained. After staining, HepG2 cells were fixed with 1 mL of 4% paraformaldehyde solution for 15 minutes. Fluorescence images of mCherry protein expression were acquired by confocal microscopy using a filter set with an excitation wavelength of 587 nm and an emission wavelength of 610 nm.

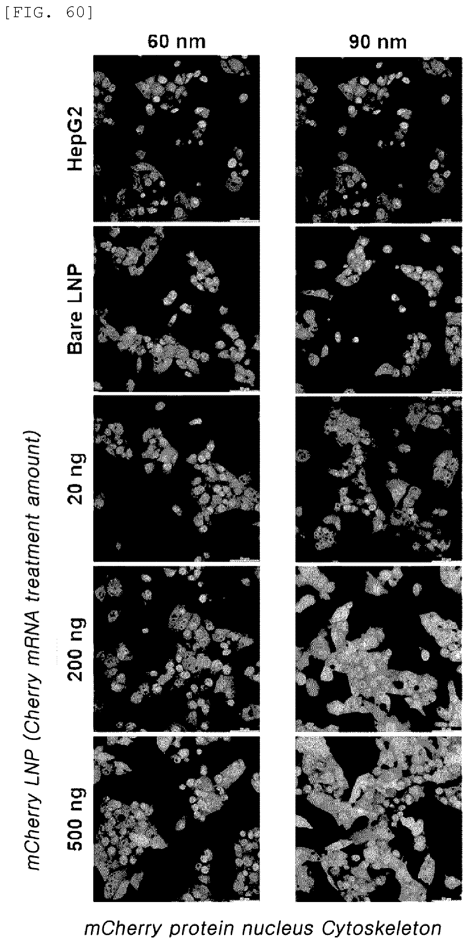

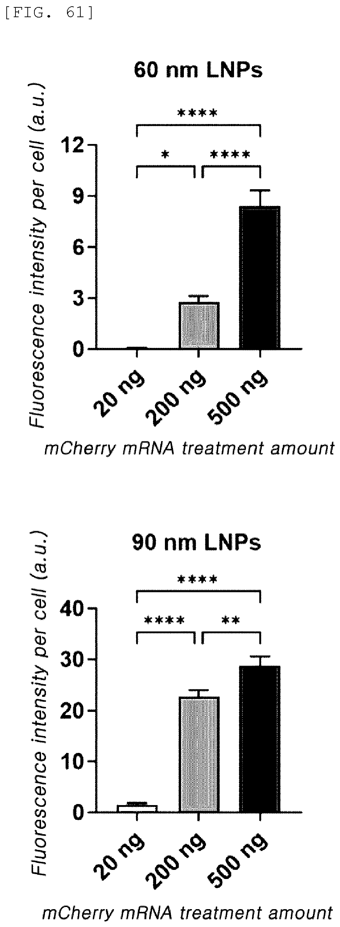

The level of mCherry protein expression in HepG2 cells following treatment with different concentrations of mCherry mRNA was evaluated using the fluorescence images shown in FIG. 60. Treatment with mCherry LNPs confirmed cytoplasmic expression of mCherry protein, as evidenced by the overlap of cytoskeletal fluorescence (green) and mCherry fluorescence (red). Protein expression increased proportionally with the amount of mRNA administered (20, 200 or 500 ng), regardless of LNP size (60 or 90 nm). Furthermore, as shown in the quantitative results of FIG. 61, at the same mRNA dose, larger LNPs yielded higher mCherry protein expression than smaller LNPs.

To evaluate protein expression in response to different mRNA LNP concentrations, THP-1 human monocyte cells were treated with mCherry LNPs synthesized at 60 and 90 nm. Cells were incubated with 20, 200 or 500 ng of each LNP for 24 h under 5% $CO_2$ at 37 °C. After removing residual LNPs from the culture medium, the cells were further cultured for 24 h, followed by replacement of the medium and staining of the nucleus and cytoskeleton. The cells were then fixed with 1 mL of 4% paraformaldehyde for 15 min. mCherry protein expression in fixed cells was visualized by confocal microscopy using a filter set with an excitation wavelength of 587 nm and an emission wavelength of 610 nm.

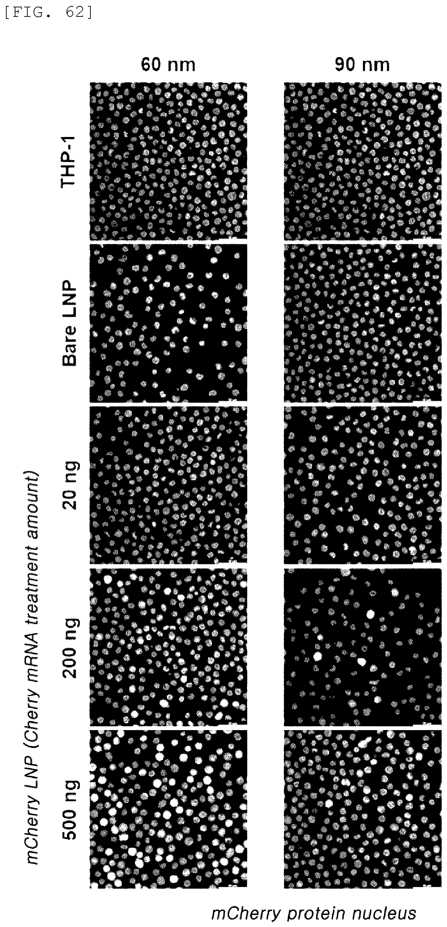

As shown in FIG. 62, the degree of mCherry protein expression in THP-1 cells depending on concentration-dependent mCherry mRNA treatment was confirmed through fluorescence imaging. When THP-1 cells were treated with mCherry LNP, the expression of mCherry protein in the cytoplasmic region was confirmed by the overlap of the fluorescence (green) staining the cytoskeleton and the fluorescence of mCherry (red).

When THP-1 cells were treated with 60 nm mCherry LNPs, the amount of mCherry protein expressed increased proportionally as the amount of administered mCherry mRNA (20, 200 500 ng) increased. In contrast, treatment with 90 nm mCherry LNPs did not show a proportional relationship between the amount of administered mCherry mRNA and the level of mCherry protein expression in THP-1 cells.

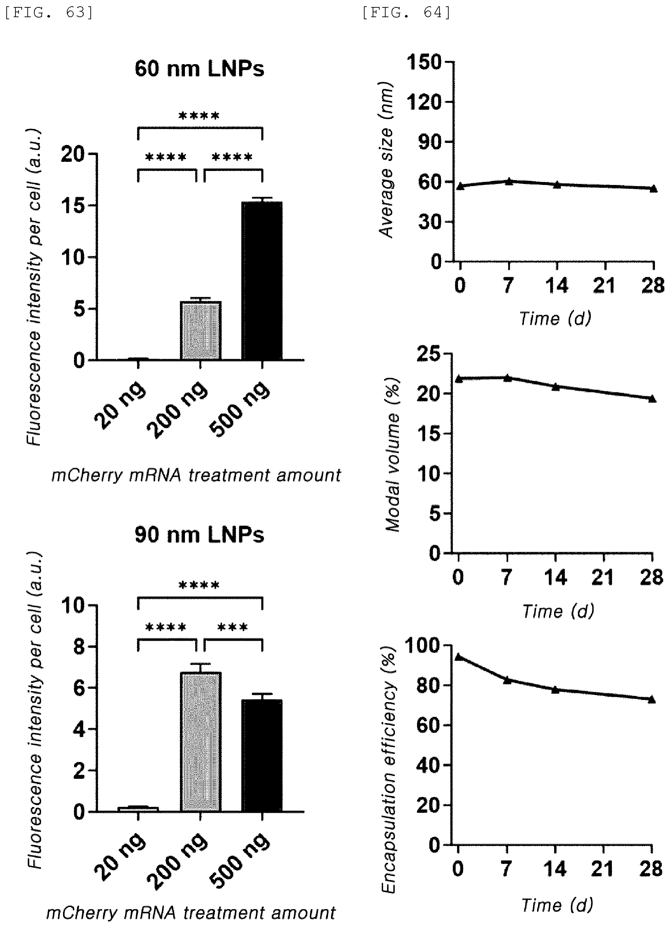

Further, when comparing the level of mCherry expression according to the size of mCherry LNP (60 nm or 90 nm) when treating the same amount of mCherry mRNA, it was confirmed through the fluorescence image in FIG. 62 and the quantitative results in FIG. 63 that 60 nm mCherry LNP showed higher mCherry expression than 90 nm mCherry LNP.

In the present invention, it was confirmed that the larger the size of mCherry LNP, the higher the mCherry protein expression in both HepG2 as an epithelial cell and U87MG as an epithelial-like cell. This may be because the content of PEGylated lipid (mol%) increases under the condition in which the LNP size becomes smaller, so that the intracellular uptake or endosomal escape of LNP is delayed due to the increase in PEG contained in the LNP.

Further, in human immune cells (THP-1), it was confirmed that mCherry protein expression was higher when treated with relatively small 60 nm mCherry LNPs rather than with large 90 nm mCherry LNPs, unlike epithelial or epithelial-like cells. This result suggests that a specific LNP size exists to efficiently deliver nucleic acids depending on the cell type to be targeted.

**[Example 12]**

**Stability verification of nucleic acid-containing LNPs**

In order to verify the stability of nucleic acid-containing LNPs, the size-specific LNP synthesis conditions set in Table 17 of Example 8-3 were applied using the microfluidic device used in Examples 8, 9 and 10, and nucleic acid-containing LNPs were synthesized by preparing a citrate buffer solution at pH 3.0 such that the aqueous solution contained nucleic acids (mRNA or oligonucleotides) at an N/P ratio of 6.

To confirm the storage stability of mCherry LNP and Cas9 LNP, two sizes were synthesized for each type of mRNA (mCherry mRNA and Cas9 mRNA) and compared. mCherry LNP was synthesized to have sizes of 60 and 90 nm, and Cas9 LNP was synthesized to have sizes of 30 and 90 nm. The synthesized mCherry LNP and Cas9 LNP were stored in PBS containing 1% trehalose, and the storage stability for 0, 7, 14 and 28 days under refrigerated conditions at 4 °C was confirmed through DLS results (mean size, size at the highest peak of the number-weighted size distribution, percentage at the highest peak of the volume-weighted size distribution, and PDI), nucleic acid encapsulation efficiency, and nucleic acid loading efficiency.

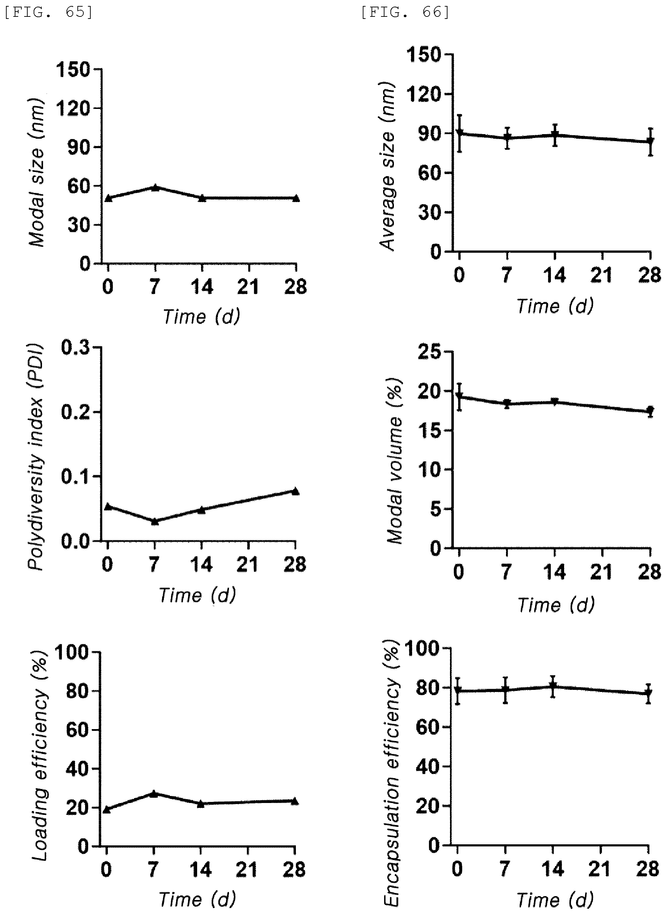

As shown in FIGS. 64 and 65, it was confirmed that the 60 nm mCherry LNP maintained an average size and the highest peak of the number-weighted size distribution without significant variation for 28 days under refrigerated conditions at 4 °C. Accordingly, the percentage (volume %) at the highest peak in the volume-weighted size distribution, which indicates the fraction of the main size in the size distribution within the nanoparticles, remained about 20% without significant change, and the PDI, which indicates uniformity, was also maintained within 0.1, confirming that it remained very uniform even after 28 days. The nucleic acid encapsulation efficiency decreased from 100% on day 0 to 82% on day 7, and to 73% on day 28. The nucleic acid loading efficiency was maintained at about 20% for 28 days.

As shown in FIGS. 66 and 67, 90 nm mCherry LNP showed similar results in terms of size to the 60 nm mCherry LNP under refrigerated conditions at 4 °C. It was confirmed that the average size and the size at the highest peak of the number-weighted size distribution remained without significant variation for 28 days. Accordingly, the percentage (volume %) at the highest peak in the volume-weighted size distribution, which indicates the fraction of the main size in the size distribution within the nanoparticles, also remained at 15% to 20% without significant change, and the PDI, which indicates the uniformity, was also maintained within 0.1, confirming that it remained very uniform even after 28 days. The nucleic acid encapsulation efficiency showed a different aspect compared with the 60 nm mCherry LNP, and was confirmed to be maintained at approximately 80% for 28 days, while the nucleic acid loading efficiency was maintained at approximately 40% for 28 days.

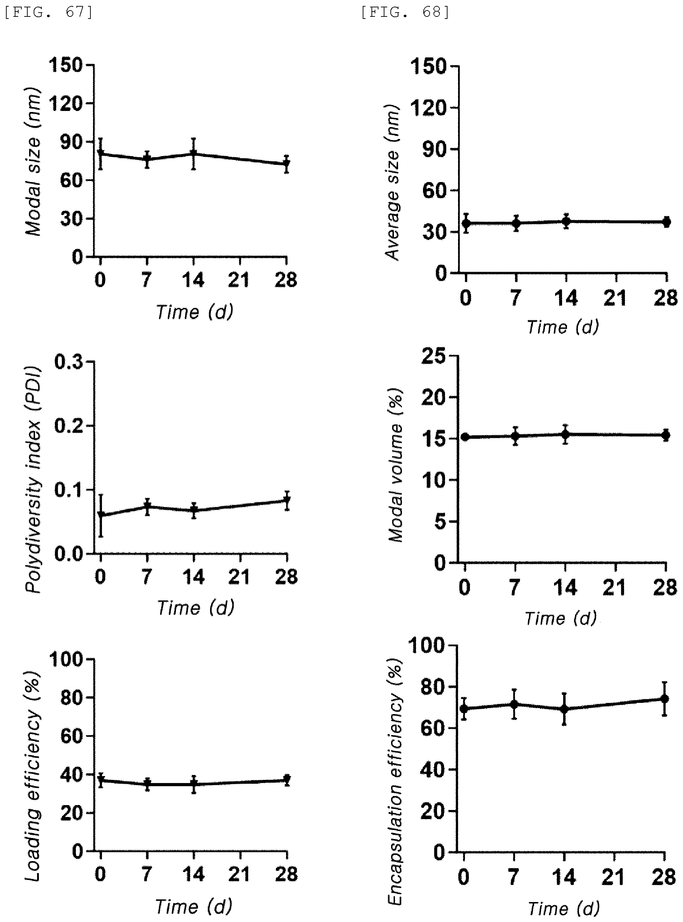

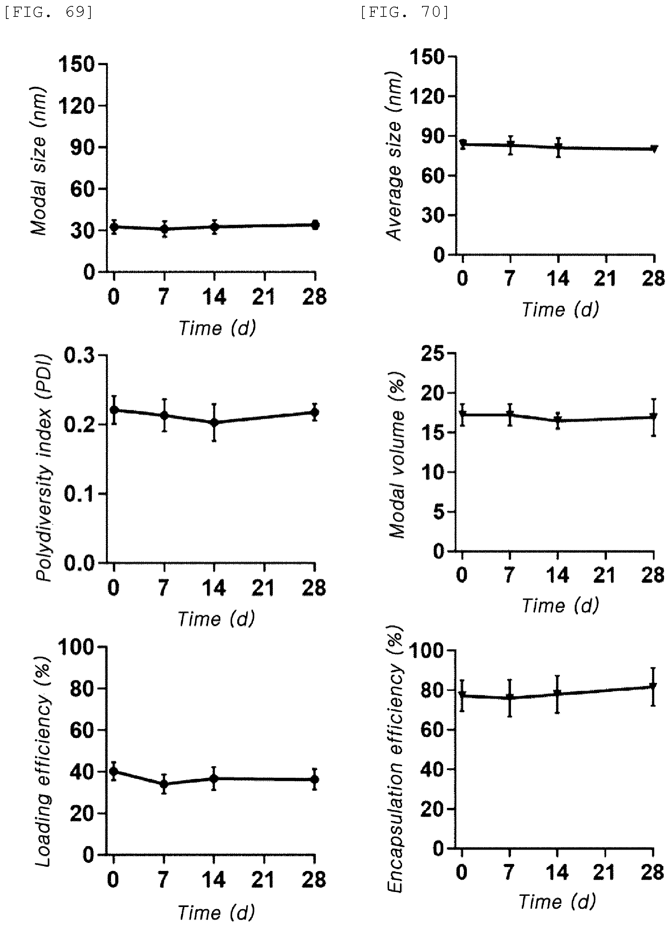

As shown in FIGS. 68 and 69, it was confirmed that the 30 nm Cas9 LNP maintained the average size and the highest peak of the number-weighted size distribution without significant variation for 28 days under refrigerated conditions at 4 °C. Accordingly, the percentage (volume %) at the highest peak of the volume-weighted size distribution, which indicates the fraction of the main size in the size distribution within the nanoparticles, was maintained at about 15% without significant variation. Further, the PDI, which indicates uniformity, was measured to be 0.22, and it was confirmed that the uniformity was also retained by maintaining a value in the 0.2 range without significant variation in PDI for 28 days. Since the PDI generally considered to indicate poor nanoparticle quality is 0.7 or more, a PDI of about 0.2 still represents uniformity. It was confirmed that the nucleic acid encapsulation efficiency was maintained at 70% to 80% for 28 days despite the small size of the LNP, and the nucleic acid loading efficiency was maintained at approximately 40% for 28 days.

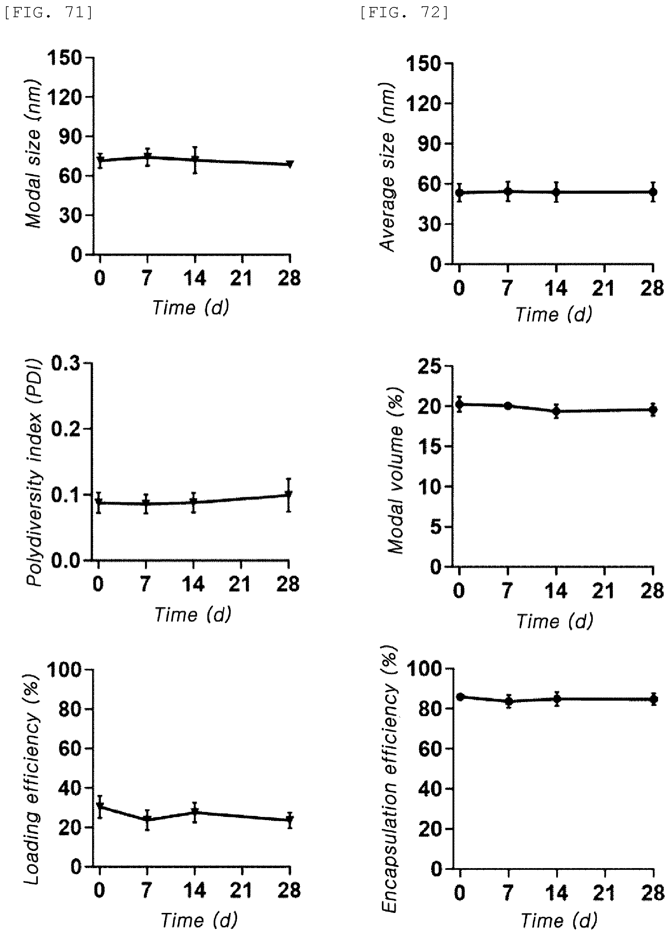

As shown in FIGS. 70 and 71, the 90 nm Cas9 LNP was confirmed to maintain the average size and the highest peak of the number-weighted size distribution without significant variation for 28 days under refrigerated conditions at 4 °C.

Accordingly, the percentage (volume %) at the highest peak of the volume-weighted size distribution, which indicates the fraction of the main size in the size distribution within the nanoparticles, remained at 15% to 20% without significant change, and the PDI, which indicates uniformity, was also maintained at about 0.1, confirming that it was very uniform even after 28 days. The nucleic acid encapsulation efficiency was confirmed to be maintained at about 80% for 28 days, and the nucleic acid loading efficiency was maintained at about 30% for 28 days.

When mRNA LNPs were synthesized using the microfluidic device of the present invention, it was confirmed that the size stability and uniformity of the LNPs were maintained for 28 days under refrigerated conditions regardless of the mRNA length or LNP size. Since the nucleic acid encapsulation efficiency was found to decrease in one type of LNP (60 nm mCherry LNP) among the four types of mRNA LNPs, it can be seen that optimization of storage conditions is required.

Oligo LNPs were synthesized in three sizes under the size-specific LNP synthesis conditions set in Table 17 of Example 8-3. As in Example 10, the resulting particles were larger than the target sizes. The sizes of Oligo LNPs synthesized with target sizes of 30, 60 and 90 nm were measured to be 50, 100 and 130 nm, respectively, showing an increase of about 20 to 40 nm compared to the target sizes. The synthesized Oligo LNPs were stored in PBS containing 1% trehalose, and their storage stability at 0, 7, 14 and 28 days under refrigerated conditions at 4 °C was evaluated by DLS (average size, size at the highest peak of the number-weighted size distribution, percentage at the highest peak of the volume-weighted size distribution (volume %), and PDI), as well as by nucleic acid encapsulation efficiency and nucleic acid loading efficiency.

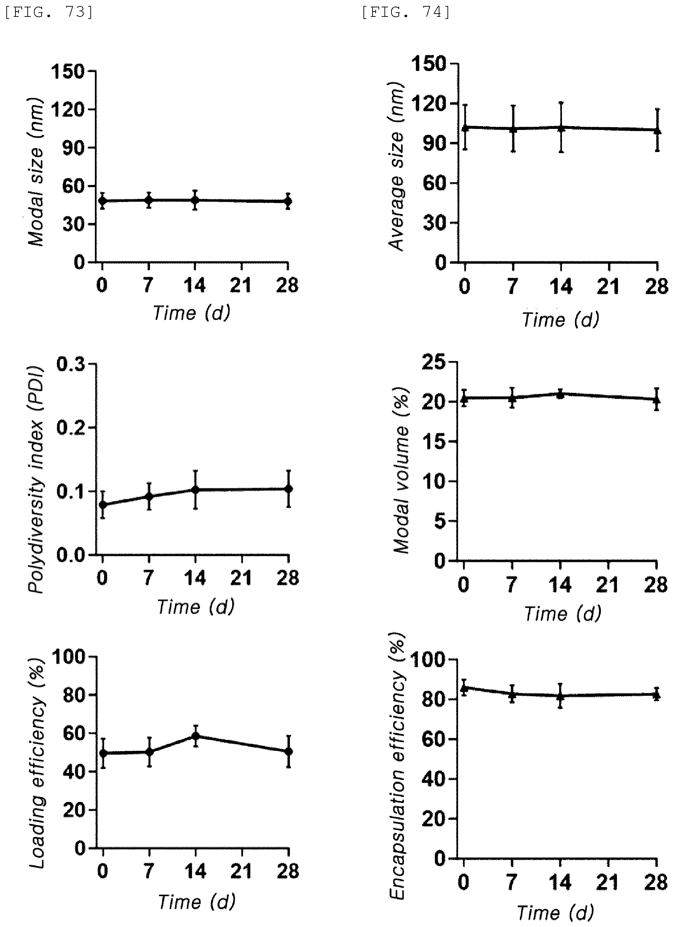

As shown in FIGS. 72 and 73, the 50 nm Oligo LNP (target size: 30 nm) maintained the average size and the highest peak of the number-weighted size distribution without significant variation for 28 days under refrigerated conditions at 4 °C. Accordingly, the percentage (volume %) at the highest peak of the volume-weighted size distribution, which indicates the fraction of the main size in the nanoparticle size distribution, remained at about 20%, and the PDI, which indicates uniformity, was also kept about 0.1, confirming that high uniformity was preserved even after 28 days. The nucleic acid encapsulation efficiency was maintained at 80% or higher for 28 days, and the nucleic acid loading efficiency was maintained at about 50% for 28 days.

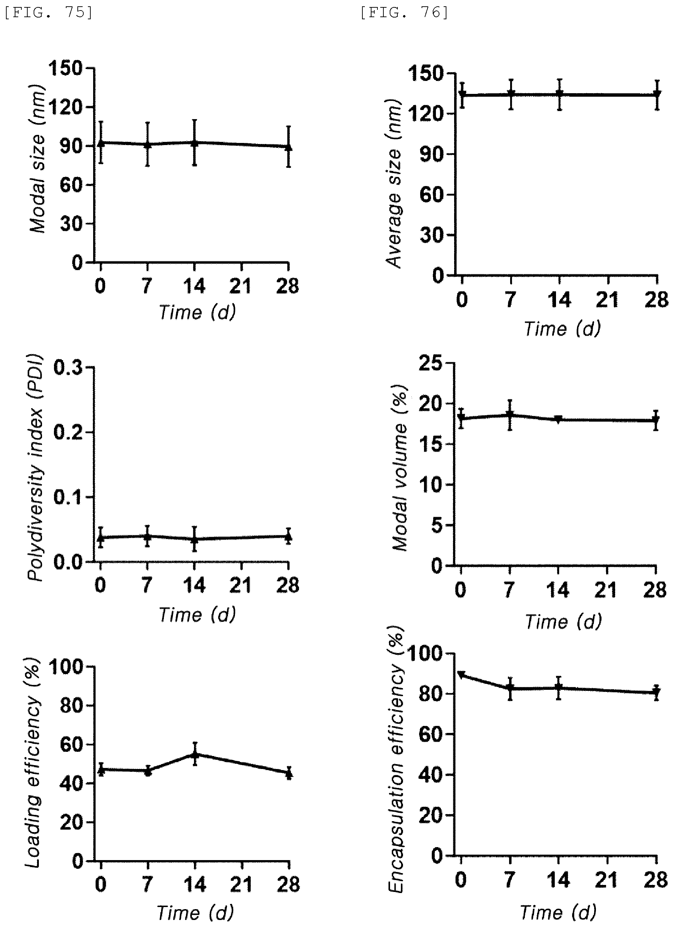

As shown in FIGS. 74 and 75, the 100 nm Oligo LNP (target size: 60 nm) maintained the average size and the highest peak of the number-weighted size distribution without significant variation for 28 days under refrigerated conditions at 4 °C. Accordingly, the percentage (volume %) at the highest peak of the volume-weighted size distribution, which represents the fraction of the main size in the nanoparticle population, remained at 20%, and the PDI, which indicates uniformity, was also kept about 0.1, confirming that high uniformity was preserved even after 28 days. The nucleic acid encapsulation efficiency was maintained at 80% or higher for 28 days, and the nucleic acid loading efficiency was maintained at about 50% for 28 days.

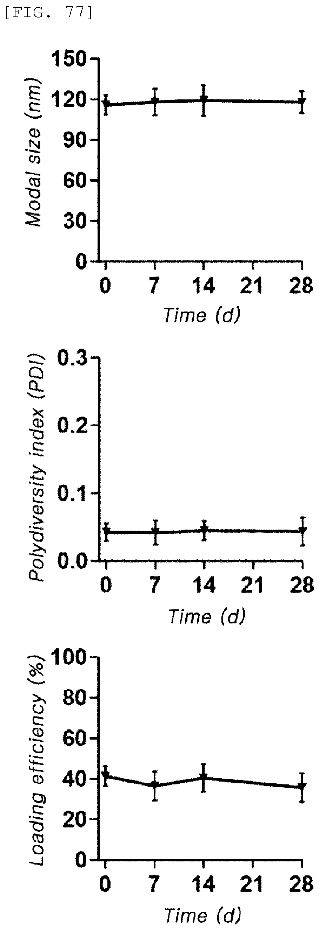

As shown in FIGS. 76 and 77, the 130 nm Oligo LNP (target size: 90 nm) maintained the average size and the highest peak of the number-weighted size distribution without significant variation for 28 days under refrigerated conditions at 4 °C. Accordingly, the percentage (volume %) at the highest peak of the volume-weighted size distribution, which represents the fraction of the main size in the nanoparticle population, remained at 15% to 20%, and the PDI, which indicates uniformity, was kept about 0.1, confirming that high uniformity was preserved even after 28 days. The nucleic acid encapsulation efficiency decreased from 89% on day 0 to 82% on day 7, but remained at 80% on day 28, while the nucleic acid loading efficiency was maintained at about 50% throughout the 28-day period.

When Oligo LNPs were synthesized using the microfluidic device of the present invention, it was confirmed that the size stability and uniformity of the LNPs were maintained for 28 days under refrigerated conditions regardless of the LNP size. In addition, it was confirmed that there was no significant change in the nucleic acid encapsulation efficiency or the nucleic acid loading efficiency.

Based on the results of the above examples, when size-specific LNPs are synthesized under three optimized conditions using the microfluidic device, LNPs of the desired size can be synthesized with very high uniformity on the basis of a high mixing efficiency of 0.95 or more, and nucleic acids of various lengths can be loaded with high nucleic acid encapsulation efficiency and nucleic acid loading efficiency. Therefore, the optimized LNP synthesis technology of the present invention is considered to enable the selection and development of LNPs of an appropriate size with high uniformity according to various indications for treatment or prevention.

## Claims

1. A microfluidic device for preparing lipid nanoparticles, comprising an inlet channel, a mixing channel, and an outlet channel,

    wherein the number of the inlet channels is two or more;
    wherein the mixing channel includes microposts;
    wherein flow directions of fluids flowing into different inlet channels are different from each other;

wherein a lipid mixture is introduced into one inlet channel, and nucleic acid is introduced into another inlet channel;

wherein the lipid mixture comprises sterol, and further comprises one or more selected from the group consisting of an ionizable lipid, a helper lipid and a PEGylated lipid;

wherein the microposts are arranged in one or more rows in the direction of the fluid flow;

wherein in one row, one or a plurality of microposts are arranged in a direction different from the direction of the fluid flow;

wherein the plurality of microposts are not arranged in a single row in the direction of the fluid flow, and arranged so as to partially or completely cover the gaps between microposts arranged in adjacent rows when viewed in the direction of the fluid flow;

wherein the Reynolds number for the fluid flow in the mixing channel is 25 to 100; and

wherein the molar ratio of sterol to the lipid mixture is 0.3 to 0.86.

2. The microfluidic device according to claim 1, wherein the lipid mixture is introduced in the same direction as the fluid flow, and the nucleic acid is introduced in a direction different from the fluid flow.

3. The microfluidic device according to claim 2, wherein the lipid mixture is introduced in the same direction as the fluid flow, and the nucleic acid is introduced in a direction perpendicular to the fluid flow.

4. The microfluidic device according to claim 1, wherein the microposts are connected or not connected to a wall forming the mixing channel.

5. The microfluidic device according to claim 1, wherein the size of the lipid nanoparticles is adjusted by the molar ratio of the lipid mixture to the sterol.

[FIG. 1]

[FIG. 2]

Unit: mm

[FIG. 3]

Forming regular symmetric micro-vortex around micropost

[FIG. 4]

[FIG. 5]

[FIG. 6]

[FIG. 7]

[FIG. 8]

[FIG. 9]

[FIG. 10]

Flow rate ratio
(Nucleic acid-containing aqueous solution:Lipid mixture)

[FIG. 11]

Residence time of a material in synthetic channel, Tres

$$\tau_{res} = \frac{\text{Channel length in fluid flow direction } (L)}{\text{Fluid line velocity } (v)}$$

[FIG. 12]

(a) Visualization of fluid flow in microfluidic device (Preparative Example 1)

Nucleic acid-containing aqueous solution

Lipid mixture

Nucleic acid-containing aqueous solution

(b) Visualization of fluid flow in microfluidic device (Preparative Example 2)

Nucleic acid-containing aqueous solution

Lipid mixture

Nucleic acid-containing aqueous solution

[FIG. 13]

| | A (기준) | B | C | D | E | F | G |
|---|---|---|---|---|---|---|---|
| **(1)** Micropost initial spacing | 2 | 1 | 4 | 2 | 2 | 2 | 2 |
| **(2)** Spacing between microposts | 0.4 | 0.4 | 0.4 | 0.2 | 0.8 | 0.4 | 0.4 |
| **(3)** Micropost width | 0.4 | 0.4 | 0.4 | 0.4 | 0.4 | 0.2 | 0.8 |

[FIG. 14]

[FIG. 15]

(Rear gap)

(Front gap)

*Mixing efficiency*

[FIG. 16]

[FIG. 17]

| Analysis table | Magnification | | | |
|---|---|---|---|---|
| | X1 | X2 | X3 | X4 |
| | -O- a) | -◐- b) | -◐- c) | -●- d) |
| Channel length (mm) | 12 | 24 | 36 | 48 |
| Channel width (mm) | 2 | 4 | 6 | 8 |
| Channel height (mm) | 0.2 | 0.2 | 0.2 | 0.2 |
| Front gap (mm) | 1 | 2 | 3 | 4 |
| Rear gap (mm) | 0.5 | 1 | 1.5 | 2 |

[FIG. 18]

[FIG. 19]

| Analysis table | Magnification | | | |
|---|---|---|---|---|
| | X1 | X2 | X3 | X4 |
| | ⊶ a) | ⬤⊶ b) | ⬤⊶ c) | ⬤⊶ d) |

[FIG. 20]

[FIG. 21]

*SM-102 : DSPC/POPC : Cholesterol : DMG-PEG2k composition ratio (molar ratio)*
**= 50:10:38.5:1.5**

[FIG. 22]

[FIG. 23]

[FIG. 24]

*SM-102:DSPC/POPC:Cholesterol:DMG-PEG2k composition ratio (molar ratio)*

**= 28:5.7:65.5:0.8**

[FIG. 25]  [FIG. 26]

[FIG. 27]

*SM-102 : DSPC/POPC : Cholesterol : DMG-PEG2k composition ratio (molar ratio)*

**= 20:4:75.5:0.5**

[FIG. 28]

[FIG. 29]

[FIG. 30]

[FIG. 31]

[FIG. 32]

Cholesterol molar ratio

[FIG. 33]　　　　　　　　　　　　　　　　[FIG. 34]

Cholesterol molar ratio

[FIG. 35]                                    [FIG. 36]

Cholesterol molar ratio

[FIG. 37]

*SM-102 : DSPC/POPC : Cholesterol : DMG-PEG2k composition ratio (molar ratio)*

**= 48.5:9.5:37.5:4.5**

[FIG. 38]

[FIG. 39]

[FIG. 40]

[FIG. 41]

[FIG. 42]

[FIG. 43]

mCherry LNP

[FIG. 44]

[FIG. 46]

[FIG. 45]

[FIG. 47]

[FIG. 48]

[FIG. 49]

[FIG. 50]

Cas9 LNP

[FIG. 52]

[FIG. 53]

[FIG. 54]

[FIG. 55]

[FIG. 56]

## Oligo LNP

[FIG. 57]

[FIG. 58]

U87MG

30 nm

60 nm

90 nm

*mCherry protein nucleus Cytoskeleton*

[FIG. 59]

[FIG. 60]

mCherry protein nucleus Cytoskeleton

[FIG. 61]

**60 nm LNPs**

Fluorescence intensity per cell (a.u.)

****
*    ****

12

9

6

3

0

20 ng    200 ng    500 ng

mCherry mRNA treatment amount

**90 nm LNPs**

Fluorescence intensity per cell (a.u.)

****
****    **

40

30

20

10

0

20 ng    200 ng    500 ng

mCherry mRNA treatment amount

[FIG. 62]

mCherry protein nucleus

[FIG. 63]

[FIG. 64]

[FIG. 65]

[FIG. 66]

[FIG. 67]

[FIG. 68]

[FIG. 69]

[FIG. 70]

[FIG. 71]

[FIG. 72]

[FIG. 73]

[FIG. 74]

[FIG. 75]

[FIG. 76]

[FIG. 77]

## INTERNATIONAL SEARCH REPORT

| International application No. |
|---|
| **PCT/KR2025/000889** |

| A. | CLASSIFICATION OF SUBJECT MATTER |
|---|---|
| | **A61K 9/51**(2006.01)i; **A61K 31/7088**(2006.01)i; **A61K 31/7105**(2006.01)i; **A61K 9/1272**(2025.01)i; **B01L 3/00**(2006.01)i; **B01J 19/00**(2006.01)i |

According to International Patent Classification (IPC) or to both national classification and IPC

| B. | FIELDS SEARCHED |
|---|---|

Minimum documentation searched (classification system followed by classification symbols)

A61K 9/51(2006.01); A61K 31/337(2006.01); B01F 5/06(2006.01); B01L 3/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 지질나노입자(lipid nanoparticles), 미세유체장치(microfluidic device), 채널(channel), 지질(lipid), 핵산(nucleic acids), 스테롤(sterol), 레이놀즈 수(reynolds number)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | MAEKI, M. et al. Microfluidic technologies and devices for lipid nanoparticle-based RNA delivery. Journal of Controlled Release. 2022, vol. 344, pp. 80–96.<br>See entire document. | 1-5 |
| A | KR 10-2614058 B1 (MEPSGEN CO., LTD.) 15 December 2023 (2023-12-15)<br>See entire document. | 1-5 |
| A | US 2018-0280970 A1 (THE UNIVERSITY OF BRITISH COLUMBIA) 04 October 2018 (2018-10-04)<br>See entire document. | 1-5 |
| A | KARAMDAD, K. et al. Studying the effects of asymmetry on the bending rigidity of lipid membranes formed by microfluidics. Chem. Commun. 2016, vol. 52, document 5277, pp. 1-4.<br>See entire document. | 1-5 |
| PX | KR 10-2716110 B1 (MEPSGEN CO., LTD.) 15 October 2024 (2024-10-15)<br>See entire document.<br>※ This document is the published patent of a priority application of the present international application. | 1-5 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 April 2025** | **14 April 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2025/000889**

| Patent document cited in search report | | | Publication date (day/month/year) | Patent family member(s) | | | Publication date (day/month/year) |
|---|---|---|---|---|---|---|---|
| KR | 10-2614058 | B1 | 15 December 2023 | WO | 2023-243865 | A1 | 21 December 2023 |
| US | 2018-0280970 | A1 | 04 October 2018 | CA | 2853316 | A1 | 02 May 2013 |
| | | | | CA | 2853316 | C | 27 November 2018 |
| | | | | EP | 2770980 | A1 | 03 September 2014 |
| | | | | EP | 2770980 | A4 | 04 November 2015 |
| | | | | EP | 3069785 | A1 | 21 September 2016 |
| | | | | EP | 3915545 | A1 | 01 December 2021 |
| | | | | JP | 2015-502337 | A | 22 January 2015 |
| | | | | JP | 2017-081954 | A | 18 May 2017 |
| | | | | JP | 2019-116478 | A | 18 July 2019 |
| | | | | JP | 2020-121986 | A | 13 August 2020 |
| | | | | JP | 2022-141709 | A | 29 September 2022 |
| | | | | JP | 2024-147535 | A | 16 October 2024 |
| | | | | JP | 7588809 | B2 | 25 November 2024 |
| | | | | US | 10843194 | B2 | 24 November 2020 |
| | | | | US | 11648556 | B2 | 16 May 2023 |
| | | | | US | 2014-0328759 | A1 | 06 November 2014 |
| | | | | US | 2016-0214103 | A1 | 28 July 2016 |
| | | | | US | 2021-0023556 | A1 | 28 January 2021 |
| | | | | US | 2023-0256436 | A1 | 17 August 2023 |
| | | | | US | 9943846 | B2 | 17 April 2018 |
| | | | | WO | 2013-059922 | A1 | 02 May 2013 |
| KR | 10-2716110 | B1 | 15 October 2024 | None | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8058069 B2

**Non-patent literature cited in the description**

- **CAMILLA, H., A et al.** The role of lipid components in lipid nanoparticles for vaccines and gene therapy. *Adv. Drug Delivery. Rev.*, 2022, vol. 188, 114416
- **CHENG, Q. et al.** Selective organ targeting (SORT) nanoparticles for tissue-specific mRNA delivery and CRISPR-Cas gene editing. *Nat. Nanotech.*, 2020, vol. 15 (4), 313-320
- **BEKARD, I. et al.** The Effects of Shear Flow on Protein Structure and Function. *Biopolymers*, 2011, vol. 95 (11), 733-745
- **VELENCIA, P et al.** Single-Step Assembly of Homogenous Lipid-Polymeric and Lipid-Quantum Dot Nanoparticles Enabled by Microfluidic Rapid Mixing. *ACS Nano 4*, 2010 (3), 1671-1679
- **RHEE, M. et al.** Drop Mixing in a Microchannel for Lab-on-a-Chip Platforms. *Langmuir*, 2008, vol. 24 (2), 590-601
- **ROHIT, K. et al.** Microfluidic Platform for Controlled Synthesis of Polymeric Nanoparticles. *Nano Letters*, 2008, vol. 8 (9), 2906-12
- **JOHNSON ; B.K. et al.** Mechanism for Rapid Self-Assembly of Block Copolymer Nanoparticles.. *Physical Review Letters*, 2003, vol. 91 (11), 118302-1, 4